# EUROPEAN PATENT APPLICATION

(11) **EP 2 476 678 A1**
(43) Date of publication of application: **18.07.2012**
(21) Application number: 10815462.6
(22) Date of filing: 10.09.2010
(51) Int. Cl.: C07D 401/14, A61K 31/444, A61P 1/04, A61P 9/12, A61P 11/00, A61P 11/06, A61P 11/08, A61P 19/00, A61P 27/06, A61P 43/00, C07D 417/14

(54) **SUBSTITUTED CARBONYL COMPOUNDS**

(30) Priority: 11.09.2009 JP 2009210792
(71) Applicant: Ube Industries, Ltd., Ube-shi Yamaguchi 755-8633 (JP)
(72) Inventor: HAGIHARA, Masahiko, Yamaguchi 755-8633 (JP); IWAMURA, Ryo, Yamaguchi 755-8633 (JP); SHIBAKAWA, Nobuhiko, Yamaguchi 755-8633 (JP); YONEDA, Kenji, Yamaguchi 755-8633 (JP); OKANARI, Eiji, Yamaguchi 755-8633 (JP); NAKANISHI, Takayuki, Yamaguchi 755-8633 (JP)
(74) Representative: Goddar, Heinz J.
(86) International application number: PCT/JP2010/065654
(87) International publication number: WO 2011/030868

(57) **Abstract**

This is to provide a novel substituted carbonyl compound represented by the following formula (I) having an excellent bronchodilatory action based on potent EP2 agonistic action and useful for treatment of respiratory diseases.

A compound represented by the following formula (I): 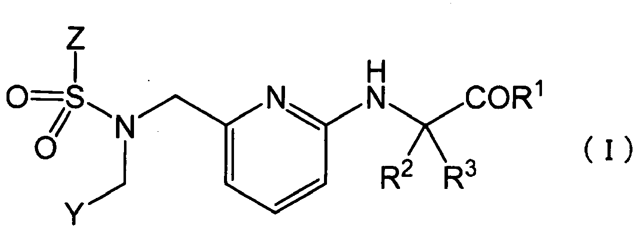 or a salt thereof

## Description

### TECHNICAL FIELD

The present invention relates to a novel substituted carbonyl compound or pharmaceutically acceptable salt thereof, that is useful as a pharmaceutical. More particularly, the substituted carbonyl compound as related to the present invention has EP2 agonistic action and is therefore useful as a therapeutic and/or prophylactic agent for respiratory diseases such as asthma or chronic obstructive pulmonary disease (hereinafter abbreviated as COPD).

### BACKGROUND ART

Prostaglandin E₂ (hereinafter abbreviated as PGE₂), which is administered by inhalation, has been reported to inhibit immediate-type and late-type asthmatic responses in asthma patients (see Non-Patent Literature 1). In addition, PGE₂ is known to act as an agonist against receptors such as EP1, EP2, EP3 and EP4, and its agonistic action against EP2 receptor in particular has been suggested to be intimately involved with bronchodilatory action (see Non-Patent Literature 2).

Sulfonamide compounds, which have a structure that resembles the compound of the present invention, have been previously found to have EP2 agonistic action (see Patent Literatures 1 to 4). In particular, the compound described as Example 14e in Patent Literature 2 has been reported to increase concentration of cyclic adenosine monophosphate (hereinafter abbreviated as cAMP) due to its EP2 agonistic action, and have an action that accelerates healing of fractures (see Non-Patent Literature 3). However, there are no specific descriptions regarding bronchodilatory action based on EP2 agonistic action of these compounds described in Patent Literatures 1 to 4, and there are no specific disclosures in any of these publications regarding a sulfonamide compound related to the present invention having the pyridylaminoacetic acid or ester therof as a partial structure.

### PRIOR ART LITERATURES

### [Patent Literatures]

[Patent Literature 1] WO98/28264 A
[Patent Literature 2] WO99/19300 A
[Patent Literature 3] WO2004/078169 A
[Patent Literature 4] WO2008/015517 A

### [Non-Patent Literatures]

[Non-Patent Literature 1] American Journal of Respiratory and Critical Care Medicine, 159,31(1999)
[Non-Patent Literature 2] American Journal of Physiology-Lung Cellular and Molecular Physiology, 284, L599 (2003)
[Non-Patent Literature 3] Proceedings of the National Academy of Sciences of the United States of America, 100, 6736 (2003)

### SUMMARY OF THE INVENTION

### PROBLEMS TO BE SOLVED BY THE INVENTION

The present inventors have carried out intensive studies on various sulfonamide compounds to develop a superior therapeutic agent or prophylactic agent for respiratory diseases, and as a result, they have found that a novel substituted carbonyl compound having a specific structure has superior bronchodilatory action based on potent EP2 agonistic action of its active form, while also having superior properties in terms of tissue distribution, bioavailability (BA), fast-acting pharmacological effect, sustained pharmacological effect, solubility, physical stability, drug interaction, toxicity and the like, and is particularly useful as a therapeutic and/or prophylactic agent (and preferably a therapeutic agent) for respiratory diseases such as asthma or COPD, thereby leading to completion of the present invention.
The present invention is to provide a novel substituted carbonyl compound or a pharmaceutically acceptable salt thereof, that has superior bronchodilatory action based on potent EP2 agonistic action, and is particularly useful as a therapeutic and/or prophylactic agent (and preferably a therapeutic agent) for respiratory diseases such as asthma or COPD.

### MEANS TO SOLVE THE PROBLEMS

The "substituted carbonyl compound" in the present invention means a compound represented by the following formula (I): 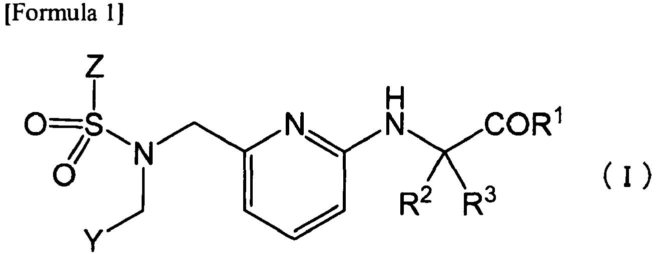 [wherein
R¹ represents either one of the following (a) to (c);
   (a) a group -OR⁵ (wherein R⁵ represents a C₇-C₂₂ alkyl group; a C₇-C₁₈ aralkyl group; a halogeno-C₁-C₆ alkyl group; a C₃-C₈ cycloalkyl group which may be substituted by a group(s) selected from the group consisting of a halogeno group, an oxo group, a C₁-C₆ alkyl group, a halogeno-C₁-C₆ alkyl group and a C₁-C₅ alkylene group; a C₁-C₆ alkyl group which is substituted by a group(s) selected from the group consisting of a C₃-C₈ cycloalkyl group, a C₂-C₆ alkanoyloxy group, an N,N-di(C₁-C₆ alkyl)aminocarbonyl group, a (C₁-C₆ alkoxy)carbonyloxy group and a 5- to 6-membered heterocyclic group),
   (b) a group -O(CH₂CH_{2O})ₘR⁶ (wherein R⁶ represents a hydrogen atom or a benzyl group, m is an integer of 1 to 4.), or
   (c) a group -NR⁷R⁸ (wherein R⁷ and R⁸ may be the same or different from each other, and each represents a hydrogen atom, a C₁-C₁₂ alkyl group, a halogeno-C₁-C₆ alkyl group or a C₃-C₈ cycloalkyl group, or, R⁷ and R⁸ may form a 4- to 8-membered ring in combination which may be substituted by a group(s) selected from the group consisting of a halogeno group, an oxo group, a C₁-C₆ alkyl group, a halogeno-C₁-C₆ alkyl group and a C₁-C₅ alkylene group.),
R² and R³ each independently represent a hydrogen atom or a C₁-C₆ alkyl group,
Y represents either one of the following (d) to (g);
   (d) a bicyclic heteroaromatic ring group which may be substituted by the same or different 1 to 5 group(s) selected from the group consisting of a halogeno group, a C₁-C₆ alkyl group, a halogeno-C₁-C₆ alkyl group, a C₁-C₆ alkoxy group, a halogeno-C₁-C₆ alkoxy group and a C₁-C₆ alkylthio group,
   (e) a group -Q₁-Q₂ (wherein Q₁ represents an arylene group or 5- to 6-membered heteroarylene group, Q² represents an aryl group or 5- to 6-membered heterocyclic group each of which may be substituted by the same or different 1 to 5 group(s) selected from the group consisting of a halogeno group, a hydroxy group, a C₁-C₆ alkyl group, a halogeno-C₁-C₆ alkyl group, a C₁-C₆ alkoxy group and a halogeno-C₁-C₆ alkoxy group.),
   (f) an aryl group or a 5- to 6-membered heterocyclic group which is substituted by the group represented by the formula (II): 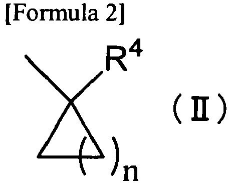 [wherein R⁴ represents a C₁-C₁₂ alkyl group or a C₁-C₆ alkoxy group, and n is an integer of 1 to 4.], or
   (g) an aryl group or a 5- to 6-membered heterocyclic group each of which may be substituted by the same or different 1 to 5 group(s) selected from the group consisting of a C₁-C₈ alkyl group, a halogeno-C₁-C₆ alkyl group, a C₁-C₆ alkoxy group, a halogeno-C₁-C₆ alkoxy group and a C₂-C₆ alkenyl group,
      Z represents an aryl group or a 5 to 6-membered heteroaromatic group each of which may be substituted by a group(s) selected from the group consisting of a halogeno group, a C₁-C₆ alkyl group, a halogeno-C₁-C₆ alkyl group, a C₁-C₆ alkoxy group and a halogeno-C₁-C₆ alkoxy group.]
or a pharmaceutically acceptable salt thereof

### EFFECTS OF THE INVENTION

The substituted carbonyl compound represented by the formula (I) or a pharmaceutically acceptable salt thereof of the present invention demonstrates superior bronchodilatory action based on potent EP2 agonistic action of its active form, and also has superior properties in terms of tissue distribution, bioavailability (BA), fast-acting pharmaceutically effect, sustained pharmacological effect, solubility, physical stability, drug interaction, toxicity and the like. Thus, the present invention is able to provide a novel compound having superior properties as a therapeutic and/or prophylactic agent for respiratory diseases (such as asthma, COPD, bronchitis, emphysema, pulmonary fibrosis, acute respiratory distress syndrome (ARDS), cystic fibrosis and pulmonary hypertension). Moreover, the compound represented by the formula (1) of the present invention is also useful as a therapeutic and/or prophylactic agent for diseases for which EP2 agonistic action is thought to be useful (such as bone diseases, gastric ulcer, hypertension and glaucoma).

### EMBODIMENT TO CARRY OUT THE INVENTION

In the compound represented by the above-mentioned formula (I), the "C₁-C₆ alkyl group" shown by the respective substituents or the "C₁-C₆ alkyl group" portion in the respective substituents each means a "C₁-C₆ alkyl group" having the same meanings, and such a "C₁-C₆ alkyl group" may be mentioned, for example, a linear or branched C₁-C₆ alkyl group such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1-ethylpropyl group, a 1,2-dimethylpropyl group, a hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethyl-1-methylpropyl group, a 1-ethyl-2-methylpropyl group, a 1,1,2-trimethylpropyl group and a 1,2,2-trimethylpropyl group, etc., preferably a C₁-C₄ alkyl group, more preferably a methyl group, an ethyl group, a propyl group, an isopropyl group or a tert-butyl group, and particularly preferably a methyl group or an ethyl group.

The "halogeno-C₁-C₆ alkyl group" shown by the respective substituents means a "halogeno-C₁-C₆ alkyl group" each having the same meanings, and such a "halogeno-C₁-C₆ alkyl group" may be mentioned, for example, a linear or branched halogeno-C₁-C₆ alkyl group such as a trifluoromethyl group, a difluoromethyl group, a fluoromethyl group, a trichloromethyl group, a dichloromethyl group, a chloromethyl group, a pentafluoroethyl group, a 2,2,2-trifluoroethyl group, a 2-fluoroethyl group, a 2,2,2-trichloroethyl group, a 2-chloroethyl group, a 2-bromoethyl group, a heptafluoropropyl group, a 3,3,3-trifluoropropyl group, a 3-fluoropropyl group, a 3-chloropropyl group, a 1,2,2,2-tetrafluoro-1-trifluoromethylethyl group, a 2,2,2-trifluoro-1-methylethyl group, a 2-fluoro-1-methylethyl group, a 2-chloro-1-methylethyl group, a perfluorobutyl group, a 4,4,4-trifluorobutyl group, a 4-fluorobutyl group, a 4-chlorobutyl group, a perfluoro-tert-butyl group, a 2,2,2-trifluoro-1,1-dimethylethyl group, a 2-fluoro-1,1-dimethylethyl group, a 2-chloro-1,1-dimethylethyl group, a perfluoropentyl group and a perfluorohexyl group, etc., preferably a halogeno-C₁-C₄ alkyl group, more preferably a trifluoromethyl group, a difluoromethyl group, a trichloromethyl group, a dichloromethyl group, a 2,2,2-trifluoroethyl group or a 2,2,2-trichloroethyl group, and particularly preferably a trifluoromethyl group or a 2,2,2-trifluoroethyl group.

The "C₁-C₁₂ alkyl group" shown by the respective substituents means a "C₁-C₁₂ alkyl group" each having the same meanings, and such a "C₁-C₁₂ alkyl group" may be mentioned, for example, a linear or branched C₁-C₁₂ alkyl group such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a 1-ethylpropyl group, a hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 2,2-dimethylbutyl group, a 3,3-dimethylbutyl group, a 1,3-dimethylbutyl group, a 2,3-dimethylbutyl group, a heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 1,2-dimethylpentyl group, a 1,3-dimethylpentyl group, a 1,4-dimethylpentyl group, a 2,3-dimethylpentyl group, a 2,4-dimethylpentyl group, a 3,4-dimethylpentyl group, a 1,1-dimethylpentyl group, a 2,2-dimethylpentyl group, a 3,3-dimethylpentyl group, a 4,4-dimethylpentyl group, a 1-methyl-2-ethylbutyl group, an octyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 1,1-dimethylhexyl group, a 2,2-dimethylhexyl group, a 3,3-dimethylhexyl group, a 4,4-dimethylhexyl group, a 5,5-dimethylhexyl group, a 1-propylpentyl group, a 2-propylpentyl group, a nonyl group, a 1-methyloctyl group, a 2-methyloctyl group, a 3-methyloctyl group, a 4-methyloctyl group, a 5-methyloctyl group, a 6-methyloctyl group, a 7-methyloctyl group, a 1-ethylheptyl group, a 2-ethylheptyl group, a 3-ethylheptyl group, a 4-ethylheptyl group, a 5-ethylheptyl group, a 1,1-dimethylheptyl group, a 2,2-dimethylheptyl group, a 3,3-dimethylheptyl group, a 4,4-dimethylheptyl group, a 5,5-dimethylheptyl group, a 1-propylhexyl group, a 2-propylhexyl group, a 3-propylhexyl group, a decyl group, a 1-methylnonyl group, a 2-methylnonyl group, a 3-methylnonyl group, a 4-methylnonyl group, a 5-methylnonyl group, a 6-methylnonyl group, a 7-methylnonyl group, a 8-methylnonyl group, a 1,1-dimethyloctyl group, a 2,2-dimethyloctyl group, a 3,3-dimethyloctyl group, a 4,4-dimethyloctyl group, a 5,5-dimethyloctyl group, a 6,6-dimethyloctyl group, a 1-ethyloctyl group, a 2-ethyloctyl group, a 3-ethyloctyl group, a 4-ethyloctyl group, a 5-ethyloctyl group, a 6-ethyloctyl group, a 1-propylheptyl group, a 2-propylheptyl group, a 3-propylheptyl group, a 4-propylheptyl group, an undecyl group, a 1-methyldecyl group, a 2-methyldecyl group, a 3-methyldecyl group, a 4-methyldecyl group, a 5-methyldecyl group, a 6-methyldecyl group, a 7-methyldecyl group, a 8-methyldecyl group, a 9-methyldecyl group, a 1,1-dimethylnonyl group, a 2,2-dimethylnonyl group, a 3,3-dimethylnonyl group, a 4,4-dimethylnonyl group, a 5,5-dimethylnonyl group, a 6,6-dimethylnonyl group, a 7,7-dimethylnonyl group, a 8,8-dimethylnonyl group, a 1-ethylnonyl group, a 2-ethylnonyl group, a 3-ethylnonyl group, a 4-ethylnonyl group, a 5-ethylnonyl group, a 6-ethylnonyl group, a 7-ethylnonyl group, a 1-propyloctyl group, a 2-propyloctyl group, a 3-propyloctyl group, a 4-propyloctyl group, a 5-propyloctyl group, a dodecyl group, a 1-methylundecyl group, a 2-methylundecyl group, a 3-methylundecyl group, a 4-methylundecyl group, a 5-methylundecyl group, a 6-methylundecyl group, a 7-methylundecyl group, a 8-methylundecyl group, a 9-methylundecyl group, a 10-methylundecyl group, a 1,1-dimethyldecyl group, a 2,2-dimethyldecyl group, a 3,3-dimethyldecyl group, a 4,4-dimethyldecyl group, a 5,5-dimethyldecyl group, a 6,6-dimethyldecyl group, a 7,7-dimethyldecyl group, a 8,8-dimethyldecyl group, a 9,9-dimethyldecyl group, a 1-ethyldecyl group, a 2-ethyldecyl group, a 3-ethyldecyl group, a 4-ethyldecyl group, a 5-ethyldecyl group, a 6-ethyldecyl group, a 7-ethyldecyl group, a 8-ethyldecyl group, a 1-propylnonyl group, a 2-propylnonyl group, a 3-propylnonyl group, a 4-propylnonyl group, a 5-propylnonyl group or a 6-propylnonyl group, etc., preferably a C₁-C₁₁ alkyl group, more preferably a methyl group, an ethyl group, a propyl group, an isopropyl group, a butyl group, an isobutyl group, a sec-butyl group, a tert-butyl group, a pentyl group, a hexyl group or an undecyl group, and particularly preferably a methyl group, an ethyl group, a propyl group, an isopropyl group, a butyl group, a hexyl group or an undecyl group.

The "C₁-C₈ alkyl group" which is a substituent(s) for the aryl group or 5- to 6-membered heterocyclic group shown by Y means the same meanings as the C₁-C₈ alkyl group contained in the above-mentioned "C₁-C₁₂ alkyl group", preferably a C₃-C₆ alkyl group, more preferably a propyl group, an isopropyl group, a butyl group, a tert-butyl group, a neopentyl group, a tert-pentyl group, a 1-ethylpropyl group or a 1-ethyl-1-methylpropyl group, particularly preferably a tert-butyl group, a neopentyl group, a tert-pentyl group, a 1-ethylpropyl group or a 1-ethyl-1-methylpropyl group.

The "C₇-C₂₂ alkyl group" shown by R⁵ in the group -OR⁵ represented by R¹, may be mentioned, for example, a linear or branched C₇-C₂₂ alkyl group such as a heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 1,2-dimethylpentyl group, a 1,3-dimethylpentyl group, a 1,4-dimethylpentyl group, a 2,3-dimethylpentyl group, a 2,4-dimethylpentyl group, a 3,4-dimethylpentyl group, a 1,1-dimethylpentyl group, a 2,2-dimethylpentyl group, a 3,3-dimethylpentyl group, a 4,4-dimethylpentyl group, a 1-methyl-2-ethylbutyl group, an octyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 1,1-dimethylhexyl group, a 2,2-dimethylhexyl group, a 3,3-dimethylhexyl group, a 4,4-dimethylhexyl group, a 5,5-dimethylhexyl group, a 1-propylpentyl group, a 2-propylpentyl group, a nonyl group, a 1-methyloctyl group, a 2-methyloctyl group, a 3-methyloctyl group, a 4-methyloctyl group, a 5-methyloctyl group, a 6-methyloctyl group, a 7-methyloctyl group, a 1-ethylheptyl group, a 2-ethylheptyl group, a 3-ethylheptyl group, a 4-ethylheptyl group, a 5-ethylheptyl group, a 1,1-dimethylheptyl group, a 2,2-dimethylheptyl group, a 3,3-dimethylheptyl group, a 4,4-dimethylheptyl group, a 5,5-dimethylheptyl group, a 1-propylhexyl group, a 2-propylhexyl group, a 3-propylhexyl group, a decyl group, a 1-methylnonyl group, a 2-methylnonyl group, a 3-methylnonyl group, a 4-methylnonyl group, a 5-methylnonyl group, a 6-methylnonyl group, a 7-methylnonyl group, a 8-methylnonyl group, a 1,1-dimethyloctyl group, a 2,2-dimethyloctyl group, a 3,3-dimethyloctyl group, a 4,4-dimethyloctyl group, a 5,5-dimethyloctyl group, a 6,6-dimethyloctyl group, a 1-ethyloctyl group, a 2-ethyloctyl group, a 3-ethyloctyl group, a 4-ethyloctyl group, a 5-ethyloctyl group, a 6-ethyloctyl group, a 1-propylheptyl group, a 2-propylheptyl group, a 3-propylheptyl group, a 4-propylheptyl group, an undecyl group, a 1-methyldecyl group, a 2-methyldecyl group, a 3-methyldecyl group, a 4-methyldecyl group, a 5-methyldecyl group, a 6-methyldecyl group, a 7-methyldecyl group, a 8-methyldecyl group, a 9-methyldecyl group, a 1,1-dimethylnonyl group, a 2,2-dimethylnonyl group, a 3,3-dimethylnonyl group, a 4,4-dimethylnonyl group, a 5,5-dimethylnonyl group, a 6,6-dimethylnonyl group, a 7,7-dimethylnonyl group, a 8,8-dimethylnonyl group, a 1-ethylnonyl group, a 2-ethylnonyl group, a 3-ethylnonyl group, a 4-ethylnonyl group, a 5-ethylnonyl group, a 6-ethylnonyl group, a 7-ethylnonyl group, a 1-propyloctyl group, a 2-propyloctyl group, a 3-propyloctyl group, a 4-propyloctyl group, a 5-propyloctyl group, a dodecyl group, a 1-methylundecyl group, a 2-methylundecyl group, a 3-methylundecyl group, a 4-methylundecyl group, a 5-methylundecyl group, a 6-methylundecyl group, a 7-methylundecyl group, a 8-methylundecyl group, a 9-methylundecyl group, a 10-methylundecyl group, a 1,1-dimethyldecyl group, a 2,2-dimethyldecyl group, a 3,3-dimethyldecyl group, a 4,4-dimethyldecyl group, a 5,5-dimethyldecyl group, a 6,6-dimethyldecyl group, a 7,7-dimethyldecyl group, a 8,8-dimethyldecyl group, a 9,9-dimethyldecyl group, a 1-ethyldecyl group, a 2-ethyldecyl group, a 3-ethyldecyl group, a 4-ethyldecyl group, a 5-ethyldecyl group, a 6-ethyldecyl group, a 7-ethyldecyl group, a 8-ethyldecyl group, a 1-propylnonyl group, a 2-propylnonyl group, a 3-propylnonyl group, a 4-propylnonyl group, a 5-propylnonyl group, a 6-propylnonyl group, a tridecyl group, a tetradecyl group, a pentadecyl group, a cetyl group, a heptadecyl group, an octadecyl group, a nonadecyl group, an eicosyl group, a heneicosyl group and a docosyl group, etc., preferably a heptyl group, an octyl group, a nonyl group, a decyl group, an undecyl group, a dodecyl group, a tridecyl group, a tetradecyl group, a pentadecyl group, a cetyl group, a heptadecyl group, an octadecyl group, a nonadecyl group, an eicosyl group, a heneicosyl group or a docosyl group, and particularly preferably an undecyl group or a docosyl group.

The "C₃-C₈ cycloalkyl group" shown by the respective substituents means a "C₃-C₈ cycloalkyl group" each having the same meanings, and such a "C₃-C₈ cycloalkyl group" may be mentioned, for example, a C₃-C₈ cycloalkyl group such as a cyclopropyl group, a cyclobutyl group, a cyclopentyl group, a cyclohexyl group, a cycloheptyl group and a cyclooctyl group, etc., preferably a cyclopropyl group, a cyclobutyl group, a cyclopentyl group or a cyclohexyl group, more preferably a cyclopropyl group, a cyclobutyl group or a cyclohexyl group, and particularly preferably a cyclopropyl group or a cyclohexyl group.

The "C₂-C₆ alkenyl group" which is a substituent(s) for the aryl group or 5- to 6-membered heterocyclic group shown by Y may be mentioned, for example, a linear or branched C₂-C₆ alkenyl group such as a vinyl group, a 1-propenyl group, a 2-propenyl group, an isopropenyl group, a 1-butenyl group, a 2-butenyl group, a 3-butenyl group, a 1-methyl-1-propenyl group, a 2-methyl-1-propenyl group, a 1-methyl-2-propenyl group, a 2-methyl-2-propenyl group, a 1-ethylvinyl group, a 1-pentenyl group, a 2-pentenyl group, a 3-pentenyl group, a 4-pentenyl group, a 1-methyl-1-butenyl group, a 2-methyl-1-butenyl group, a 3-methyl-1-butenyl group, a 1-methyl-2-butenyl group, a 2-methyl-2-butenyl group, a 3-methyl-2-butenyl group, a 1-methyl-3-butenyl group, a 2-methyl-3-butenyl group, a 3-methyl-3-butenyl group, a 1-ethyl-1-propenyl group, a 1-methyl-2-propenyl group, a 1,1-dimethyl-2-propenyl group, a 1-hexenyl group, a 2-hexenyl group, a 3-hexenyl group, a 4-hexenyl group, a 5-hexenyl group, a 1-methyl-1-pentenyl group, a 2-methyl-1-pentenyl group, a 3-methyl-1-pentenyl group, a 4-methyl-1-pentenyl group, a 1-methyl-2-pentenyl group, a 2-methyl-2-pentenyl group, a 3-methyl-2-pentenyl group, a 4-methyl-2-pentenyl group, a 1-methyl-3-pentenyl group, a 2-methyl-3-pentenyl group, a 3-methyl-3-pentenyl group, a 4-methyl-3-pentenyl group, a 1-methyl-4-pentenyl group, a 2-methyl-4-pentenyl group, a 3-methyl-4-pentenyl group, a 4-methyl-4-pentenyl group, a 1-ethyl-1-butenyl group, a 2-ethyl-1-butenyl group, a 1-ethyl-2-butenyl group, a 2-ethyl-2-butenyl group, a 1-ethyl-3-butenyl group, a 2-ethyl-3-butenyl group, a 1,1-dimethyl-2-butenyl group and a 1,1-dimethyl-3-butenyl group, etc., preferably a C₄-C₆ alkenyl group, more preferably a 1-methyl-1-propenyl group, a 1-methyl-1-butenyl group or a 1-methyl-1-pentenyl group, and particularly preferably a 1-methyl-1-pentenyl group.

The "C₁-C₆ alkoxy group" shown by the respective substituents or the "C₁-C₆ alkoxy group" portion in the respective substituents each means a "C₁-C₆ alkoxy group" having the same meanings, and such a "C₁-C₆ alkoxy group" may be mentioned, for example, a linear or branched C₁-C₆ alkoxy group such as a methoxy group, an ethoxy group, a propoxy group, an isopropoxy group, a butoxy group, an isobutoxy group, a sec-butoxy group, a tert-butoxy group, a pentyloxy group, an isopentyloxy group, a neopentyloxy group, a tert-pentyloxy group, a 1-methylbutoxy group, a 2-methylbutoxy group, a 1-ethylpropoxy group, a 1,2-dimethylpropoxy group, a hexyloxy group, a 1-methylpentyloxy group, a 2-methylpentyloxy group, a 3-methylpentyloxy group, a 4-methylpentyloxy group, a 1-ethylbutoxy group, a 2-ethylbutoxy group, a 1,1-dimethylbutoxy group, a 1,2-dimethylbutoxy group, a 1,3-dimethylbutoxy group, a 2,2-dimethylbutoxy group, a 2,3-dimethylbutoxy group, a 3,3-dimethylbutoxy group, a 1-ethyl-1-methylpropoxy group, a 1-ethyl-2-methylpropoxy group, a 1,1,2-trimethylpropoxy group or a 1,2,2-trimethylpropoxy group, etc., preferably a C₁-C₄ alkoxy group, more preferably a methoxy group, an ethoxy group, a propoxy group or an isopropoxy group, and particularly preferably a methoxy group.

The "halogeno-C₁-C₆ alkoxy group" shown by the respective substituents each means a "halogeno-C₁-C₆ alkoxy group" having the same meanings, and such a "halogeno-C₁-C₆ alkoxy group" may be mentioned, for example, a linear or branched halogeno-C₁-C₆ alkoxy group such as a trifluoromethoxy group, a difluoromethoxy group, a trichloromethoxy group, a dichloromethoxy group, a pentafluoroethoxy group, a 2,2,2-trifluoroethoxy group, a 2-fluoroethoxy group, a 2,2,2-trichloroethoxy group, a 2-chloroethoxy group, a 2-bromoethoxy group, a heptafluoropropoxy group, a 3,3,3-trifluoropropoxy group, a 3-fluoropropoxy group, a 3-chloropropoxy group, a 1,2,2,2-tetrafluoro-1-trifluoromethylethoxy group, a 2,2,2-trifluoro-1-methylethoxy group, a 2-fluoro-1-methylethoxy group, a 2-chloro-1-methylethoxy group, a perfluorobutoxy group, a 4,4,4-trifluorobutoxy group, a 4-fluorobutoxy group, a 4-chlorobutoxy group, a perfluoro-tert-butoxy group, a 2,2,2-trifluoro-1,1-dimethylethoxy group, a 2-fluoro-1,1-dimethylethoxy group, a 2-chloro-1,1-dimethylethoxy group, a perfluoropentyloxy group and a perfluorohexyloxy group, etc., preferably a halogeno-C₁-C₄ alkoxy group, more preferably a trifluoromethoxy group, a difluoromethoxy group, a trichloromethoxy group, a dichloromethoxy group or a 2,2,2-trifluoroethoxy group, and particularly preferably a difluoromethoxy group.

The "C₂-C₆ alkanoyl group" portion of the C₂-C₆ alkanoyloxy group which is a substituent(s) for the C₁-C₆ alkyl group shown by R⁵ in the group -OR⁵ represented by R¹, there may be mentioned, for example, a linear or branched C₂-C₆ alkanoyl group such as an acetyl group, a propanoyl group, a butanoyl group, a 2-methylpropanoyl group, a pentanoyl group, a 2-methylbutanoyl group, a 3-methylbutanoyl group, a pivaloyl group, a hexanoyl group, a 2-methylpentanoyl group, a 3-methylpentanoyl group, a 4-methylpentanoyl group, a 2-ethylbutanoyl group, a 2,2-dimethylbutanoyl group, a 2,3-dimethylbutanoyl group and a 3,3-dimethylbutanoyl group, etc., preferably a C₂-C₅ alkanoyl group, more preferably an acetyl group, a propanoyl group, a butanoyl group or a pivaloyl group, and particularly preferably an acetyl group, a propanoyl group or a pivaloyl group.

The "C₇-C₁₈ aralkyl group" shown by R⁵ in the group -OR⁵ represented by R¹, there may be mentioned, for example, a benzyl group, a 1-phenylethyl group, a 2-phenylethyl group, a 1-phenylpropyl group, a 2-phenylpropyl group, a 3-phenylpropyl group, a phenylbutyl group, a phenylpentyl group, a phenylhexyl group, a phenylheptyl group, a phenyloctyl group, a phenylnonyl group, a phenyldecyl group, a phenylundecyl group, a phenyldodecyl group, a naphthalen-1-ylmethyl group, a naphthalen-2-ylmethyl group, a 1-(naphthalen-1-yl)ethyl group, a 2-(naphthalen-1-yl)ethyl group, a 1-(naphthalen-2-yl)ethyl group, a 2-(naphthalen-2-yl)ethyl group, a 1-(naphthalen-1-yl)propyl group, a 2-(naphthalen-1-yl)propyl group, a 3-(naphthalen-1-yl)propyl group, a 1-(naphthalen-2-yl)propyl group, a 2-(naphthalen-2-yl)propyl group, a 3-(naphthalen-2-yl)propyl group, a naphthalen-1-ylbutyl group, a naphthalen-2-ylbutyl group, a naphthalen-1-ylpentyl group, a naphthalen-2-ylpentyl group, a naphthalen-1-ylhexyl group, a naphthalen-2-ylhexyl group, a naphthalen-1-ylheptyl group, a naphthalen-2-ylheptyl group, a naphthalen-1-yloctyl group or a naphthalen-2-yloctyl group, etc., preferably a C₈-C₁₈ aralkyl group, more preferably a 2-phenylethyl group, a 3-phenylpropyl group, a 4-phenylbutyl group, a 5-phenylpentyl group, a 6-phenylhexyl group, a 7-phenylheptyl group, a 8-phenyloctyl group, a 9-phenylnonyl group, a 10-phenyldecyl group, a 11-phenylundecyl group or a 12-phenyldodecyl group, and particularly preferably a 5-phenylpentyl group or a 10-phenyldecyl group.

The "C₁-C₅ alkylene group" which is a substituent(s) for the C₃-C₈ cycloalkyl group shown by R⁵ in the group -OR⁵ represented by R¹, may be mentioned, for example, a methylene group, an ethylene group, propylene group, a butylene group or a pentylene group, etc., preferably a methylene group, an ethylene group or a propylene group.
The carbon atoms to which the C₁-C₅ alkylene group is bonded may be the same, or, may not be the same. The C₃-C₈ cycloalkyl group substituted by such a C₁-C₅ alkylene group may be mentioned, for example, a cycloalkyl group having a spiro structure such as a spiro[2.3]hexyl group, a spiro[2.4]heptyl group, a spiro[2.5]octyl group and a spiro[3.5]nonyl group, etc., or, a cycloalkyl group having a bicyclic structure such as a bicyclo[3.1.0]hexyl group, a bicyclo[4.1.0]heptyl group and a bicyclo[3.2.0]heptyl group, etc., preferably a spiro[2.3]hexyl group, a spiro[2.5]octyl group, a bicyclo[3.1.0]hexyl group or a bicyclo[4.1.0]heptyl group.

"C₁-C₅ alkylene group" which is a substituent(s) for the 4 to 8-membered ring formed by R⁷ and R⁸ in combination, in the group -NR⁷R⁸ represented by R¹, may be mentioned, for example, a methylene group, an ethylene group, a propylene group, a butylene group or a pentylene group, etc., preferably a methylene group, an ethylene group or a propylene group.
The carbon atom to which the C₁-C₅ alkylene group is bonded may be the same, or, may not be the same. The 4 to 8-membered ring substituted by such a C₁-C₅ alkylene group, may be mentioned, for example, an azacycloalkyl group having a spiro structure such as a 5-azaspiro[2.3]hexanyl group and 6-azaspiro[2.5]octanyl group, etc., or, an azacycloalkyl group having a bicyclic structure such as a 3-aza[3.1.0]hexanyl group, etc.

The "arylene group" shown by Q¹ in the group -Q¹-Q² represented by Y, may be mentioned, for example, a phenylene group or a naphthylene group, etc., and preferably a phenylene group.

The "aryl group" shown by the respective substituents each means an "aryl group" having the same meanings, and such an "aryl group" may be mentioned, for example, a phenyl group or a naphthyl group, etc., and preferably a phenyl group.

The "5- to 6-membered heteroarylene group" shown by Q¹ in the group -Q¹-Q² represented by Y, may be mentioned, for example, a furylene group, a thienylene group, a thiazolylene group, a pyridylene group, a pyridazinylene group or a pyrimidinylene group, etc., preferably a thienylene group, a pyridazinylene group or a pyrimidinylene group, and particularly preferably a thienylene group.

The "bicyclic heteroaromatic group" shown by Y may be mentioned, for example, a benzofuryl group, a benzothienyl group, a benzoxazolyl group, a benzothiazolyl group, an isoindolyl group, an indolyl group, an indazolyl group, a benzimidazolyl group, an isoquinolyl group or a quinolyl group, etc., preferably a benzofuryl group, a benzothienyl group, a benzoxazolyl group or a benzothiazolyl group, and particularly preferably a benzofuryl group or a benzothienyl group.

The "5- to 6-membered heterocyclic group" shown by the respective substituents mean a completely unsaturated, partially unsaturated or completely saturated 5-to 6-membered cyclic group each containing 1 to 4 hetero atoms (in case of a plural number, each independently) selected from the group consisting of an oxygen atom, a nitrogen atom and a sulfur atom as a constitutional element(s) of the ring. The completely unsaturated 5- to 6-membered heterocyclic group may be mentioned, for example, a pyrrolyl group, a furyl group, a thienyl group, a pyrazolyl group, an imidazolyl group, an oxazolyl group, a thiazolyl group, a pyridyl group, a pyridazinyl group, a pyrimidinyl group or a pyradinyl group, etc., the partially unsaturated 5- to 6-membered heterocyclic group may be mentioned, for example, a 4,5-dihydro-1H-imidazolyl group, a 4,5-dihydroxazolyl group, a 4,5-dihydrothiazolyl group, a 1,4,5,6-tetrahydropyrimidinyl group, a 5,6-dihydro-4H-1,3-oxazinyl group or a 5,6-dihydro-4H-1,3-thiazinyl group, etc., and the completely saturated 5- to 6-membered heterocyclic group may be mentioned, for example, a pyrrolidinyl group, a tetrahydrofuryl group, a 1,3-dioxolanyl group, a piperidinyl group, a tetrahydropyranyl group, a piperazinyl group, a morpholinyl group, a thiomorpholinyl group, a 1,3-dioxanyl group or a 1,4-dioxanyl group, etc. The "5- to 6-membered heterocyclic group" shown by the respective substituents may be preferably mentioned a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group, a piperidinyl group or a morpholinyl group, more preferably a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group or a morpholinyl group, and particularly preferably a pyrazolyl group, a thiazolyl group, a 1,2,4-triazolyl group, a pyridyl group, a pyridazinyl group, a pyrimidinyl group, a 4,5-dihydrothiazolyl group or a morpholinyl group.
In the 5- to 6-membered heterocyclic group, the carbon atom(s) on the ring may be substituted by a C₁-C₆ alkyl group or a phenyl group, and in the partially unsaturated or completely saturated 5- to 6-membered cyclic group, the carbon atom(s) on the ring may be substituted by an oxo group. Such a group may be mentioned, for example, a 5-methyl-2-oxo-1,3-dioxolen-4-yl group or a 5-phenyl-2-oxo-1,3-dioxolen-4-yl group, etc.

The "5 to 6-membered heteroaromatic group" shown by Z means the same meanings as the above-mentioned "completely unsaturated 5- to 6-membered heterocyclic group", and may be mentioned, for example, a pyrrolyl group, a furyl group, a thienyl group, a pyrazolyl group, an imidazolyl group, an oxazolyl group, a thiazolyl group, a pyridyl group, a pyridazinyl group, a pyrimidinyl group or a pyradinyl group, etc., preferably a thienyl group, an imidazolyl group, a thiazolyl group, a pyridyl group or a pyrimidinyl group, more preferably a thienyl group or a pyridyl group, and particularly preferably a pyridyl group.

The "halogeno group" shown by the respective substituents, or the "halogeno" portion in the respective substituents each means "a halogeno group" having the same meanings, and such a "halogeno group" may be mentioned, for example, a fluoro group, a chloro group, a bromo group or an iodo group, preferably a fluoro group, a chloro group or a bromo group.

In the following formula (II): 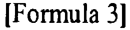 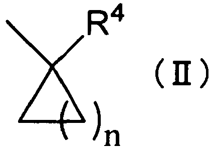 which is a substituent(s) for the aryl group or 5- to 6-membered heterocyclic group represented by Y, n is an integer of 1 to 4, particularly preferably 1 to 2.

In the group -O(CH₂CH₂O)ₘR⁶ represented by R¹, m is an integer of 1 to 4, particularly preferably 3 to 4.

The number of the substituent(s) of the bicyclic heteroaromatic ring group which may be substituted by the same or different 1 to 5 group(s) selected from the group consisting of a halogeno group, a C₁-C₆ alkyl group, a halogeno-C₁-C₆ alkyl group, a C₁-C₆ alkoxy group, a halogeno-C₁-C₆ alkoxy group and a C₁-C₆ alkylthio group, and the group -Q¹-Q² (wherein Q¹ represents an arylene group or a 5- to 6-membered heteroarylene group, and Q² represents an aryl group or a 5- to 6-membered heterocyclic group each of which may be substituted by the same or different 1 to 5 group(s) selected from the group consisting of a halogeno group, a hydroxy group, a C₁-C₆ alkyl group, a halogeno-C₁-C₆ alkyl group, a C₁-C₆ alkoxy group and a halogeno-C₁ - C₆ alkoxy group.), and, the aryl group or the 5- to 6-membered heterocyclic group each of which may be substituted by the same or different 1 to 5 group(s) selected from the group consisting of a C₁-C₈ alkyl group, a halogeno-C₁-C₆ alkyl group, a C₁-C₆ alkoxy group, a halogeno-C₁-C₆ alkoxy group and a C₂-C₆ alkenyl group, represented by Y, and, the aryl group or the 5- to 6-membered heteroaromatic group which may be substituted by a group(s) selected from the group consisting of a halogeno group, a C₁-C₆ alkyl group, a halogeno-C₁-C₆ alkyl group, a C₁-C₆ alkoxy group and a halogeno-C₁-C₆ alkoxy group, represented by Z is preferably unsubstituted or a substituent's number of 1 to 3, particularly preferably unsubstituted or a substituent's number of 1 to 2.

R¹ is preferably a group -OR⁵ (wherein R⁵ represents a C₇-C₂₂ alkyl group; a C₇-C₁₈ aralkyl group; a halogeno-C₁-C₄ alkyl group; a C₃-C₆ cycloalkyl group which may be substituted by a group(s) selected from the group consisting of a halogeno group, a C₁-C₄ alkyl group, a halogeno-C₁-C₄ alkyl group and a C₁-C₃ alkylene group; or a C₁-C₆ alkyl group which is substituted by a group(s) selected from the group - consisting of a C₃-C₆ cycloalkyl group, a C₂-C₄ alkanoyloxy group, an N,N-di(C₁-C₄ alkyl)aminocarbonyl group, a (C₁-C₄ alkoxy)carbonyloxy group and a 5- to 6-membered heterocyclic group.), a group -O(CH₂CH₂O)ₘR⁶ (wherein R⁶ represents a hydrogen atom or a benzyl group, m is an integer of 3 to 4.), or, a group -NR⁷R⁸ (wherein R⁷ and R⁸ may be the same or different from each other, and each represents a hydrogen atom, a C₁-C₁₂ alkyl group, a halogeno-C₁-C₄ alkyl group or a C₃-C₆ cycloalkyl group, or, R⁷ and R⁸ may form a 4- to 6-membered ring in combination which may be substituted by a group(s) selected from the group consisting of a halogeno group, a C₁-C₄ alkyl group, a halogeno-C₁-C₄ alkyl group and a C₁-C₃ alkylene group.), more preferably a group -OR⁵ (wherein R⁵ represents a C₇-C₂₂ alkyl group; a C₈-C₁₈ aralkyl group; a fluoro C₁-C₄ alkyl group; a C₃-C₆ cycloalkyl group which may be substituted by a group(s) selected from the group consisting of a fluoro group, a methyl group and a trifluoromethyl group; or a C₁-C₄ alkyl group which is substituted by a group(s) selected from the group consisting of a cyclopropyl group, an acetoxy group, a pivaloyloxy group, an N,N-di(C₁-C₄ alkyl)aminocarbonyl group, a methoxycarbonyloxy group, a morpholinyl group, a 5-methyl-2-oxo-1,3-dioxolen-4-yl group and a 5-phenyl-2-oxo-1,3-dioxolen-4-yl group.), a group -O(CH₂CH₂)ₘR⁶ (wherein R⁶ represents a hydrogen atom or a benzyl group, m is an integer of 3 to 4.), or, a group -NR⁷R⁸ (wherein R⁷ and R⁸ may be the same or different from each other, and each represents a hydrogen atom, a C₁-C₁₁ alkyl group, a fluoro-C₁-C₄ alkyl group or a C₃-C₆ cycloalkyl group.), further more preferably a heptyloxy group, an octyloxy group, a nonyloxy group, a decyloxy group, an undecyloxy group, a dodecyloxy group, a tridecyloxy group, a tetradecyloxy group, a pentadecyloxy group, a cetyloxy group, a heptadecyloxy group, an octadecyloxy group, a nonadecyloxy group, an eicosyloxy group, a heneicosyloxy group, a docosyloxy group, a 4-phenylbutyloxy group, a 5-phenylpentyloxy group, a 6-phenylhexyloxy group, a 7-phenylheptyloxy group, a 8-phenyloctyloxy group, a 9-phenylnonyloxy group, a 10-phenyldecyloxy group, a 11-phenylundecyloxy group, a 12-phenyldodecyloxy group, a 2,2,2-trifluoroethoxy group, a cyclohexyloxy group, an acetoxymethoxy group, a pivaloyloxymethoxy group, a 2-(dimethylamino)-2-oxoethoxy group, a 2-(diethylamino)-2-oxoethoxy group, a (5-methyl-2-oxo-1,3-dioxolen-4-yl)methoxy group, a (5-phenyl-2-oxo-1,3-dioxolen-4-yl)methoxy group, a (morpholin-4-yl)methoxy group, a 2-(morpholin-4-yl)ethoxy group, a 2-[2-(2-hydroxyethoxy)ethoxy]ethoxy group, a 2-{2-[2-(benzyloxy)ethoxy]-ethoxy}ethoxy group, a methylamino group, an ethylamino group, a propylamino group, an isopropylamino group, a butylamino group, a hexylamino group, an undecylamino group, a dimethylamino group, a diethylamino group or a (2,2,2-trifluoroethyl)-amino group, and particularly preferably an undecyloxy group, a docosyloxy group, a 5-phenylpentyloxy group, a 10-phenyldecyloxy group, a 2,2,2-trifluoroethoxy group, a 2-(dimethylamino)-2-oxoethyl group, a pivaloyloxymethyl group, a (5-methyl-2-oxo-1,3-dioxolen-4-yl)methyl group, a 2-(morpholin-4-yl)ethyl group, a 2-[2-(2-hydroxy-ethoxy)ethoxy]ethoxy group, a 2-{2-[2-(benzyloxy)ethoxy]ethoxy}ethoxy group, an undecylamino group or a dimethylamino group.

R² is preferably a hydrogen atom or C₁-C₄ alkyl group, more preferably a hydrogen atom or methyl group, and particularly preferably a hydrogen atom.

R³ is preferably a hydrogen atom or C₁-C₄ alkyl group, more preferably a hydrogen atom or a methyl group, and particularly preferably a hydrogen atom.

Y is preferably a benzofuryl group, a benzothienyl group, a benzoxazolyl group or a benzothiazolyl group each of which may be substituted by a group(s) selected from the group consisting of a halogeno group, a C₁-C₄ alkyl group, a halogeno-C₁-C₄ alkyl group, a C₁-C₄ alkoxy group, a halogeno-C₁-C₄ alkoxy group and a C₁-C₄ alkylthio group, or, a group -Q¹-Q² (Q¹ represents a phenylene group, a thienylene group, a pyridazinylene group or a pyrimidinylene group, and Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group each of which may be substituted by a group(s) selected from the group consisting of a halogeno group, a hydroxy group, a C₁-C₄ alkyl group, a halogeno-C₁-C₄ alkyl group, a C₁-C₄ alkoxy group and a halogeno-C₁-C₄ alkoxy group), or, a phenyl group which is substituted by the following formula (II): 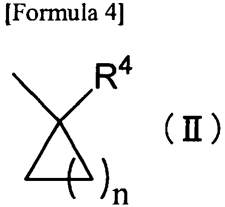 (R⁴ represents a C₁-C₁₀ alkyl group or a C₁-C₄ alkoxy group, and n is an integer of 1 to 2), or, a phenyl group or a thienyl group each of which may be substituted by a group(s) selected from the group consisting of a C₃-C₆ alkyl group, a halogeno-C₁-C₄ alkyl group, a C₁-C₄ alkoxy group, a halogeno-C₁-C₄ alkoxy group and a C₄-C₆ alkenyl group, more preferably a benzofuryl group, a benzothienyl group, a benzoxazolyl group or a benzothiazolyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group, a dichloromethoxy group, a methylthio group, an ethylthio group, a propylthio group, an isopropylthio group and a tert-butylthio group, or, a group -Q₁-Q₂ (wherein Q¹ represents a phenylene group, a thienylene group, a pyridazinylene group or a pyrimidinylene group, and Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group each of which may be substituted by a group(s) selected from the group consisting of a fluoro group, a chloro group, a bromo group, a hydroxy group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group and a dichloromethoxy group), or, a phenyl group which is substituted by a group(s) selected from the group consisting of a 1-methylcyclopropyl group, a 1-ethylcyclopropyl group, a 1-isopropylcyclopropyl group, a 1-butylcyclopropyl group, a 1-hexylcyclopropyl group, a 1-methoxycyclopropyl group and a 1-ethylcyclobutyl group, or, a phenyl group which may be substituted by a group(s) selected from the group consisting of a tert-butyl group, a neopentyl group, a tert-pentyl group, a 1-ethylpropyl group, a 1-ethyl-1-methylpropyl group, a trifluoromethyl group, a difluoromethoxy group and a 1-methyl-1-pentenyl group, further more preferably a benzofuran-2-yl group, a benzo[b]thiophen-2-yl group, a 6-chlorobenzo-[b]thiophen-2-yl group, a 6-methoxybenzo[b]thiophen-2-yl group, a biphenyl-4-yl group, a 4'-fluorobiphenyl-4-yl group, a 4'-chlorobiphenyl-4-yl group, a 4-(pyrazol-1-yl)phenyl group, a 4-(thiazol-2-yl)phenyl group, a 4-(5-chlorothiazol-2-yl)phenyl group, a 4-(5-methylthiazol-2-yl)phenyl group, a 4-(4,5-dimethylthiazol-2-yl)phenyl group, a 4-(4-trifluoromethylthiazol-2-yl)phenyl group, a 4-(thiazol-4-yl)phenyl group, a 4-(1,2,4-triazol-1-yl)phenyl group, a 4-(pyridin-2-yl)phenyl group, a 4-(pyridazin-4-yl)phenyl group, a 4-(pyrimidin-2-yl)phenyl group, a 4-(4,5-dihydrothiazol-2-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-hexylcyclopropyl)phenyl group, a 4-(1-methoxycyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)-phenyl group, a 4-(1-ethyl-1-methylpropyl)phenyl group, a 4-trifluoromethylphenyl group, a 4-difluoromethoxyphenyl group or a 4-(1-methyl-1-pentenyl)phenyl group, and particularly preferably a benzofuran-2-yl group, a benzo[b]thiophen-2-yl group, a 6-chlorobenzo[b]thiophen-2-yl group, a 6-methoxybenzo[b]thiophen-2-yl group, a biphenyl-4-yl group, a 4'-fluorobiphenyl-4-yl group, a 4-(pyrazol-1-yl)phenyl group, a 4-(thiazol-2-yl)phenyl group, a 4-(thiazol-4-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)phenyl group or a 4-(1-ethyl-1-methylpropyl)phenyl group.

Z is preferably a phenyl group, a thienyl group, an imidazolyl group, a thiazolyl group, a pyridyl group or a pyrimidinyl group each of which may be substituted by a group(s) selected from the group consisting of a halogeno group, a C₁-C₄ alkyl group, a halogeno-C₁-C₄ alkyl group, a C₁-C₄ alkoxy group and a halogeno-C₁-C₄ alkoxy group, more preferably a phenyl group, a thienyl group or a pyridyl group each of which may be substituted by a group(s) selected from the group consisting of a fluoro group, a chloro group, a methyl group, an ethyl group, a trifluoromethyl group, a methoxy group and a difluoromethoxy group, further more preferably a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-ethylphenyl group, a 4-ethyl-3-fluorophenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a thiophen-2-yl group, a thiophen-3-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group, and particularly preferably a phenyl group, a 3-fluorophenyl group, a 4-fluorophenyl group, a pyridin-2-yl group, a pyridin-3-yl group or a pyridin-4-yl group.

When there is an optical isomer, geometric isomer or rotational isomer in the compound represented by the formula (I) of the present invention, such isomers are also included in the scope of the present invention, and when there is a proton tautomer, such tautomer is also included in the present invention.

The compound represented by the formula (I) of the present invention is easily converted into a pharmaceutically acceptable salt by treating it with an acid. Examples of such a salt include, for example, inorganic acid salts such as hydrochloride, hydrobromide, hydroiodide, nitrate, sulfate and phosphate; or organic acid salts such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate and aspartate.

Further, the compound or pharmaceutically acceptable salt thereof represented by the formula (I) of the present invention can be present as a hydrate or solvate, and they are also included in the present invention.

An active form of the substituted carbonyl compound of the present invention means a compound wherein R¹ is a hydroxy group in the formula (I), and can be formed from the substituted carbonyl compound of the present invention by chemical hydrolysis, etc., or, by metabolism due to a hydrolase, etc., in a living body of a warm-blooded animal.

The compound represented by the formula (I) of the present invention is, preferably,
(1) a compound wherein R¹ is a group -OR⁵ (wherein R⁵ is a C₇-C₂₂ alkyl group; a C₇-C₁₈ aralkyl group; a halogeno-C₁-C₄ alkyl group; a C₃-C₆ cycloalkyl group which may be substituted by a group(s) selected from the group consisting of a halogeno group, a C₁-C₄ alkyl group, a halogeno-C₁-C₄ alkyl group and a C₁-C₃ alkylene group; a C₁-C₆ alkyl group which is substituted by a group(s) selected from the group consisting of a C₃-C₆ cycloalkyl group, a C₂-C₄ alkanoyloxy group, an N,N-di(C₁-C₄ alkyl)aminocarbonyl group, a (C₁-C₄ alkoxy)carbonyloxy group and a 5- to 6-membered heterocyclic group.), a group -O(CH₂CH₂O)ₘR⁶ (wherein R⁶ represents a hydrogen atom or a benzyl group, and m is an integer of 3 to 4.), or, a group -NR⁷R⁸ (wherein R⁷ and R⁸ may be the same or different from each other and each represents a hydrogen atom, a C₁-C₁₂ alkyl group, a halogeno-C₁-C₄ alkyl group or a C₃-C₆ cycloalkyl group, or, R⁷ and R⁸ may form a 4- to 6-membered ring in combination which may be substituted by a group(s) selected from the group consisting of a halogeno group, a C₁-C₄ alkyl group, a halogeno-C₁-C₄ alkyl group and a C₁-C₃ alkylene group.),
(2) a compound wherein R¹ is a group -OR⁵ (wherein R⁵ is a C₇-C₂₂ alkyl group; a C₈-C₁₈ aralkyl group; a fluoro-C₁-C₄ alkyl group; a C₃-C₆ cycloalkyl group which may be substituted by a group(s) selected from the group consisting of a fluoro group, a methyl group, a trifluoromethyl group; or a C₁-C₄ alkyl group which is substituted by a group(s) selected from the group consisting of a cyclopropyl group, an acetoxy group, a pivaloyloxy group, an N,N-di(C₁-C₄ alkyl)aminocarbonyl group, a methoxycarbonyloxy group, a morpholinyl group, a 5-methyl-2-oxo-1,3-dioxolen-4-yl group and a 5-phenyl-2-oxo-1,3-dioxolen-4-yl group.), a group -O(CH₂CH₂O)ₘR⁶ (wherein R⁶ is a hydrogen atom or a benzyl group, and m is an integer of 3 to 4.), or, a group -NR⁷R⁸ (wherein R⁷ and R⁸ may be the same or different from each other, and each represents a hydrogen atom, a C₁-C₁₁ alkyl group, a fluoro-C₁-C₄ alkyl group or a C₃-C₆ cycloalkyl group.)
(3) a compound wherein R¹ is a heptyloxy group, an octyloxy group, a nonyloxy group, a decyloxy group, an undecyloxy group, a dodecyloxy group, a tridecyloxy group, a tetradecyloxy group, a pentadecyloxy group, a cetyloxy group, a heptadecyloxy group, an octadecyloxy group, a nonadecyloxy group, an eicosyloxy group, a heneicosyloxy group, a docosyloxy group, a 4-phenylbutyloxy group, a 5-phenylpentyloxy group, a 6-phenylhexyloxy group, a 7-phenylheptyloxy group, a 8-phenyloctyloxy group, a 9-phenylnonyloxy group, a 10-phenyldecyloxy group, a 11-phenylundecyloxy group, a 12-phenyldodecyloxy group, a 2,2,2-trifluoroethoxy group, a cyclohexyloxy group, an acetoxymethoxy group, a pivaloyloxymethoxy group, a 2-(dimethylamino)-2-oxoethoxy group, a 2-(diethylamino)-2-oxoethoxy group, a (5-methyl-2-oxo-1,3-dioxolen-4-yl)-methoxy group, a (5-phenyl-2-oxo-1,3-dioxolen-4-yl)methoxy group, a (morpholin-4-yl)methoxy group, a 2-(morpholin-4-yl)ethoxy group, a 2-[2-(2-hydroxyethoxy)-ethoxy]ethoxy group, a 2-{2-[2-(benzyloxy)ethoxy]ethoxy}ethoxy group, a methylamino group, an ethylamino group, a propylamino group, an isopropylamino group, a butylamino group, a hexylamino group, an undecylamino group, a dimethylamino group, a diethylamino group or a (2,2,2-trifluoroethyl)amino group,
(4) a compound wherein R¹ is an undecyloxy group, a docosyloxy group, a 5-phenylpentyloxy group, a 10-phenyldecyloxy group, a 2,2,2-trifluoroethoxy group, a 2-(dimethylamino)-2-oxoethoxy group, a pivaloyloxymethoxy group, a (5-methyl-2-oxo-1,3-dioxolen-4-yl)methoxy group, a 2-(morpholin-4-yl)ethoxy group, a 2-[2-(2-hydroxyethoxy)ethoxy]ethoxy group, a 2-{2-[2-(benzyloxy)ethoxy]ethoxy}ethoxy group, an undecylamino group or a dimethylamino group,
(5) a compound wherein R² and R³ each independently represents a hydrogen atom or a C₁-C₄ alkyl group,
(6) a compound wherein R² and R³ each independently represents a hydrogen atom or a methyl group,
(7) a compound wherein R² and R³ are both hydrogen atoms,
(8) a compound wherein Y represents a benzofuryl group, a benzothienyl group, a benzoxazolyl group or a benzothiazolyl group each of which may be substituted by a substituent(s) selected from the group consisting of a halogeno group, a C₁-C₄ alkyl group, a halogeno-C₁-C₄ alkyl group, a C₁-C₄ alkoxy group, a halogeno-C₁-C₄ alkoxy group and a C₁-C₄ alkylthio group, or, a group -Q¹-Q² (Q¹ represents a phenylene group, a thienylene group, a pyridazinylene group or a pyrimidinylene group, Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group each of which may be substituted by a substituent(s) selected from the group consisting of a halogeno group, a hydroxy group, a C₁-C₄ alkyl group, a halogeno-C₁-C₄ alkyl group, a C₁-C₄ alkoxy group and a halogeno-C₁-C₄ alkoxy group), or, a phenyl group which is substituted by the group represented by the following formula (II): 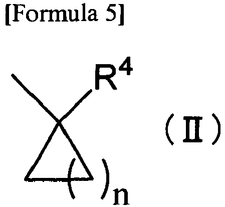 (R⁴ represents a C₁-C₁₀ alkyl group or a C₁-C₄ alkoxy group, n is an integer of 1 to 2), or, a phenyl group or a thienyl group each of which may be substituted by a group(s) selected from the group consisting of a C₃-C₆ alkyl group, a halogeno-C₁-C₄ alkyl group, a C₁-C₄ alkoxy group, a halogeno-C₁-C₄ alkoxy group and a C₄-C₆ alkenyl group,
(9) a compound wherein Y is a benzofuryl group, a benzothienyl group, a benzoxazolyl group or a benzothiazolyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group, a dichloromethoxy group, a methylthio group, an ethylthio group, a propylthio group, an isopropylthio group and a tert-butylthio group, or, a group -Q¹-Q² (wherein Q¹ represents a phenylene group, a thienylene group, a pyridazinylene group or a pyrimidinylene group, Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group each of which may be substituted by a group(s) selected from the group consisting of a fluoro group, a chloro group, a bromo group, a hydroxy group, a methyl group, an ethyl group, a propyl group, n isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group and a dichloromethoxy group), or, a phenyl group which is substituted by a group(s) selected from the group consisting of a 1-methylcyclopropyl group, a 1-ethylcyclopropyl group, a 1-isopropylcyclopropyl group, a 1-butylcyclopropyl group, a 1-hexylcyclopropyl group, a 1-methoxycyclopropyl group and a 1-ethylcyclobutyl group, or, a phenyl group which may be substituted by a group(s) selected from the group consisting of a tert-butyl group, a neopentyl group, a tert-pentyl group, a 1-ethylpropyl group, a 1-ethyl-1-methylpropyl group, a trifluoromethyl group, a difluoromethoxy group and a 1-methyl-1-pentenyl group,
(10) a compound wherein Y is a benzofuran-2-yl group, a benzo[b]thiophen-2-yl group, a 6-chlorobenzo[b]thiophen-2-yl group, a 6-methoxybenzo[b]thiophen-2-yl group, a biphenyl-4-yl group, a 4'-fluorobiphenyl-4-yl group, a 4'-chlorobiphenyl-4-yl group, a 4-(pyrazol-1-yl)phenyl group, a 4-(thiazol-2-yl)phenyl group, a 4-(5-chlorothiazol-2-yl)phenyl group, a 4-(5-methylthiazol-2-yl)phenyl group, a 4-(4,5-dimethylthiazol-2-yl)phenyl group, a 4-(4-trifluoromethylthiazol-2-yl)phenyl group, a 4-(thiazol-4-yl)-phenyl group, a 4-(1,2,4-triazol-1-yl)phenyl group, a 4-(pyridin-2-yl)phenyl group, a 4-(pyridazin-4-yl)phenyl group, a 4-(pyrimidin-2-yl)phenyl group, a 4-(4,5-dihydrothiazol-2-yl)phenyl group, a 6-phenylpyridazin-3-yl group, a 4-(1-methylcyclopropyl)-phenyl group, a 4-(1-ethylcyclopropyl)phenyl group, a 4-(1-isopropylcyclopropyl)-phenyl group, a 4-(1-butylcyclopropyl)phenyl group, a 4-(1-hexylcyclopropyl)phenyl group, a 4-(1-methoxycyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)phenyl group, a 4-(1-ethyl-1-methylpropyl)phenyl group, a 4-trifluoromethylphenyl group, a 4-difluoromethoxyphenyl group or a 4-(1-methyl-1-pentenyl)phenyl group,
(11) a compound wherein Y is a benzofuran-2-yl group, a benzo[b]thiophen-2-yl group, a 6-chlorobenzo[b]thiophen-2-yl group, a 6-methoxybenzo[b]thiophen-2-yl group, a biphenyl-4-yl group, a 4'-fluorobiphenyl-4-yl group, a 4-(pyrazol-1-yl)phenyl group, a 4-(thiazol-2-yl)phenyl group, a 4-(thiazol-4-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)phenyl group or a 4-(1-ethyl-1-methylpropyl)phenyl group,
(12) a compound wherein Z is a phenyl group, a thienyl group, an imidazolyl group, a thiazolyl group, a pyridyl group or a pyrimidinyl group, each of which may be substituted by a group selected from the group consisting of a halogeno group, a C₁-C₄ alkyl group; a halogeno-C₁-C₄ alkyl group, a C₁-C₄ alkoxy group and a halogeno-C₁-C₄ alkoxy group,
(13) a compound wherein Z is a phenyl group, a thienyl group or a pyridyl group each of which may be substituted by a group selected from the group consisting of a fluoro group, a chloro group, a methyl group, an ethyl group, a trifluoromethyl group, a methoxy group and a difluoromethoxy group,
(14) a compound wherein Z is a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-ethylphenyl group, a 4-ethyl-3-fluorophenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a thiophen-2-yl group, a thiophen-3-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group,
(15) a compound wherein Z is a phenyl group, a 3-fluorophenyl group, a 4-fluorophenyl group, a pyridin-2-yl group, a pyridin-3-yl group or a pyridin-4-yl group.

Further, in the above-mentioned groups of (1)-(4), (5)-(7), (8)-(11) and (12)-(15), as the number becomes larger, a more preferred compound is indicated, and a compound obtained by arbitrarily selecting R¹ from the groups (1)-(4), R² and R³ from the groups (5)-(7), Y from the groups (8)-(11), and Z from the groups (11)-(15), or by arbitrarily combining them is also a preferred compound.
Examples of such compound include:
(15) a compound wherein R¹ is a group -OR⁵ (wherein R⁵ is a C₇-C₂₂ alkyl group; a C₇-C₁₈ aralkyl group; a halogeno-C₁-C₄ alkyl group; a C₃-C₆ cycloalkyl group which may be substituted by a group(s) selected from the group consisting of a halogeno group, a C₁-C₄ alkyl group, a halogeno-C₁-C₄ alkyl group and a C₁-C₃ alkylene group; or a C₁-C₆ alkyl group which is substituted by a group(s) selected from the group consisting of a C₃-C₆ cycloalkyl group, a C₂-C₄ alkanoyloxy group, an N,N-di(C₁-C₄ alkyl)aminocarbonyl group, a (C₁-C₄ alkoxy)carbonyloxy group and a 5- to 6-membered heterocyclic group.), a group -O(CH₂CH₂O)ₘR⁶ (wherein R⁶ is a hydrogen atom or a benzyl group, and m is an integer of 3 to 4.), or, a group -NR⁷R⁸ (wherein R⁷ and R⁸ may be the same or different from each other, and each represents a hydrogen atom, a C₁-C₁₂ alkyl group, a halogeno-C₁-C₄ alkyl group or a C₃-C₆ cycloalkyl group, or, R⁷ and R⁸ may form a 4- to 6-membered ring in combination which may be substituted by a group(s) selected from the group consisting of a halogeno group, a C₁-C₄ alkyl group, a halogeno-C₁-C₄ alkyl group and a C₁-C₃ alkylene group.),
   R² and R³ each independently represent a hydrogen atom or a methyl group,
   Y is a benzofuryl group, a benzothienyl group, a benzoxazolyl group or a benzothiazolyl group each of which may be substituted by a group(s) selected from the group consisting of a halogeno group, a C₁-C₄ alkyl group, a halogeno-C₁-C₄ alkyl group, a C₁-C₄ alkoxy group, a halogeno-C₁-C₄ alkoxy group and a C₁-C₄ alkylthio group, or, a group -Q¹-Q² (Q¹ is a phenylene group, a thienylene group, a pyridazinylene group or a pyrimidinylene group, and Q² is a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group each of which may be substituted by a group(s) selected from the group consisting of a halogeno group, a hydroxy group, a C₁-C₄ alkyl group, a halogeno-C₁-C₄ alkyl group, a C₁-C₄ alkoxy group and a halogeno-C₁-C₄ alkoxy group), or, a phenyl group which is substituted by the following formula (II): 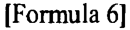 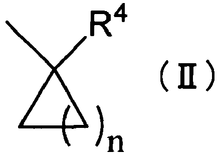 (R⁴ is a C₁-C₁₀ alkyl group or a C₁-C₄ alkoxy group, and n is an integer of 1 to 2), or, a phenyl group or a thienyl group each of which may be substituted by a group(s) selected from the group consisting of a C₃-C₆ alkyl group, a halogeno-C₁-C₄ alkyl group, a C₁-C₄ alkoxy group, a halogeno-C₁-C₄ alkoxy group and a C₄-C₆ alkenyl group,
   Z is a phenyl group, a thienyl group, an imidazolyl group, a thiazolyl group, a pyridyl group or a pyrimidinyl group each of which may be substituted by a group(s) selected from the group consisting of a halogeno group, a C₁-C₄ alkyl group, a halogeno-C₁-C₄ alkyl group, a C₁-C₄ alkoxy group and a halogeno-C₁-C₄ alkoxy group.
(16) a compound wherein R¹ is a group -OR⁵ (wherein R⁵ is a C₇-C₂₂ alkyl group; a C₈-C₁₈ aralkyl group; a fluoroC₁-C₄ alkyl group; a C₃-C₆ cycloalkyl group which may be substituted by a group(s) selected from the group consisting of a fluoro group, a methyl group, and a trifluoromethyl group; or a C₁-C₄ alkyl group which is substituted by a group(s) selected from the group consisting of a cyclopropyl group, an acetoxy group, a pivaloyloxy group, an N,N-di(C₁-C₄ alkyl)aminocarbonyl group, a methoxycarbonyloxy group, a morpholinyl group, a 5-methyl-2-oxo-1,3-dioxolen-4-yl group and a 5-phenyl-2-oxo-1,3-dioxolen-4-yl group.), a group -O(CH₂CH₂O)ₘR⁶ (wherein R⁶ is a hydrogen atom or a benzyl group, and m is an integer of 3 to 4.), or, a group -NR 7R⁸ (wherein R⁷ and R⁸ may be the same or different from each other, and each represents a hydrogen atom, a C₁-C₁₁ alkyl group, a fluoro-C₁-C₄ alkyl group or a C₃-C₆ cycloalkyl group.),
   R² and R³ each independently represent a hydrogen atom or a methyl group,
   Y is a benzofuryl group, a benzothienyl group, a benzoxazolyl group or a benzothiazolyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group, a dichloromethoxy group, a methylthio group, an ethylthio group, a propylthio group, an isopropylthio group and a tert-butylthio group, or, a group -Q¹-Q² (wherein Q¹ represents a phenylene group, a thienylene group, a pyridazinylene group or a pyrimidinylene group, and Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group each of which may be substituted by a group(s) selected from the group consisting of a fluoro group, a chloro group, a bromo group, a hydroxy group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group and a dichloromethoxy group), or, a phenyl group which is substituted by a group(s) selected from the group consisting of a 1-methylcyclopropyl group, a 1-ethylcyclopropyl group, a 1-isopropylcyclopropyl group, a 1-butylcyclopropyl group, a 1-hexylcyclopropyl group, a 1-methoxycyclopropyl group and a 1-ethylcyclobutyl group, or, a phenyl group which may be substituted by a group(s) selected from the group consisting of a tert-butyl group, a neopentyl group, a tert-pentyl group, a 1-ethylpropyl group, a 1-ethyl-1-methylpropyl group, a trifluoromethyl group, a difluoromethoxy group and a 1-methyl-1-pentenyl group, and
   Z is a phenyl group, a thienyl group or a pyridyl group each of which may be substituted by a group(s) selected from the group consisting of a fluoro group, a chloro group, a methyl group, an ethyl group, a trifluoromethyl group, a methoxy group and a difluoromethoxy group,
(17) a compound wherein R¹ is a heptyloxy group, an octyloxy group, a nonyloxy group, a decyloxy group, an undecyloxy group, a dodecyloxy group, a tridecyloxy group, a tetradecyloxy group, a pentadecyloxy group, a cetyloxy group, a heptadecyloxy group, an octadecyloxy group, a nonadecyloxy group, an eicosyloxy group, a heneicosyloxy group, a docosyloxy group, a 4-phenylbutyloxy group, a 5-phenylpentyloxy group, a 6-phenylhexyloxy group, a 7-phenylheptyloxy group, a 8 -phenyloctyloxy group, a 9-phenylnonyloxy group, a 10-phenyldecyloxy group, a 11-phenylundecyloxy group, a 12-phenyldodecyloxy group, a 2,2,2-trifluoroethoxy group, a cyclohexyloxy group, an acetoxymethoxy group, a pivaloyloxymethoxy group, a 2-(dimethylamino)-2-oxoethoxy group, a 2-(diethylamino)-2-oxoethoxy group, a (5-methyl-2-oxo-1,3-dioxolen-4-yl)methoxy group, a (5-phenyl-2-oxo-1,3-dioxolen-4-yl)methoxy group, a (morpholin-4-yl)methoxy group, a 2-(morpholin-4-yl)ethoxy group, a 2-[2-(2-hydroxyethoxy)ethoxy]ethoxy group, a 2-{2-[2-(benzyloxy)ethoxy]-ethoxy} ethoxy group, a methylamino group, an ethylamino group, a propylamino group, an isopropylamino group, a butylamino group, a hexylamino group, an undecylamino group, a dimethylamino group, a diethylamino group or a (2,2,2-trifluoroethyl)-amino group,
   R² and R³ are both hydrogen atoms,
   Y is a benzofuran-2-yl group, a benzo[b]thiophen-2-yl group, a 6-chlorobenzo-[b]thiophen-2-yl group, a 6-methoxybenzo[b]thiophen-2-yl group, a biphenyl-4-yl group, a 4'-fluorobiphenyl-4-yl group, a 4'-chlorobiphenyl-4-yl group, a 4-(pyrazol-1-yl)phenyl group, a 4-(thiazol-2-yl)phenyl group, a 4-(5-chlorothiazol-2-yl)phenyl group, a 4-(5-methylthiazol-2-yl)phenyl group, a 4-(4,5-dimethylthiazol-2-yl)phenyl group, a 4-(4-trifluoromethylthiazol-2-yl)phenyl group, a 4-(thiazol-4-yl)phenyl group, a 4-(1,2,4-triazol-1-yl)phenyl group, a 4-(pyridin-2-yl)phenyl group, a 4-(pyridazin-4-yl)-phenyl group, a 4-(pyrimidin-2-yl)phenyl group, a 4-(4,5-dihydrothiazol-2-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-hexylcyclopropyl)phenyl group, a 4-(1-methoxycyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)-phenyl group, a 4-(1-ethyl-1-methylpropyl)phenyl group, a 4-trifluoromethylphenyl group, a 4-difluoromethoxyphenyl group or a 4-(1-methyl-1-pentenyl)phenyl group, and
   Z is a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-ethylphenyl group, a 4-ethyl-3-fluorophenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a thiophen-2-yl group, a thiophen-3-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group, (18) a compound wherein R¹ is an undecyloxy group, a docosyloxy group, a 5-phenylpentyloxy group, a 10-phenyldecyloxy group, a 2,2,2-trifluoroethoxy group, a 2-(dimethylamino)-2-oxoethoxy group, a pivaloyloxymethoxy group, a (5-methyl-2-oxo-1,3-dioxolen-4-yl)methoxy group, a 2-(morpholin-4-yl)ethoxy group, a 2-[2-(2-hydroxyethoxy)ethoxy]ethoxy group, a 2-{2-[2-(benzyloxy)ethoxy]ethoxy}ethoxy group, an undecylamino group or a dimethylamino group,
   R² and R³ are both hydrogen atoms,
   Y is a benzofuran-2-yl group, a benzo[b]thiophen-2-yl group, a 6-chlorobenzo-[b]thiophen-2-yl group, a 6-methoxybenzo[b]thiophen-2-yl group, a biphenyl-4-yl group, a 4'-fluorobiphenyl-4-yl group, a 4-(pyrazol-1-yl)phenyl group, a 4-(thiazol-2-yl)phenyl group, a 4-(thiazol-4-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)phenyl group or a 4-(1-ethyl-1-methylpropyl)phenyl group, and
   Z is a phenyl group, a 3-fluorophenyl group, a 4-fluorophenyl group, a pyridin-2-yl group, a pyridin-3-yl group or a pyridin-4-yl group.
(19) The substituted carbonyl compound is preferably mentioned a substituted carbonyl compound such as
   (5-phenylpentyl) (6-'{(pyridin-2-ylsulfonyl)[4-(thiazol-2-yl)benzyl]aminomethyl}-pyridin-2-ylamino)acetate,
   (10-phenyldecyl) (6- {(pyridin-2-ylsulfonyl)[4-(thiazol-2-yl)benzyl]aminomethyl}-pyridin-2-ylamino)acetate,
   2-(6- {(Pyridin-2-ylsulfonyl)[4-(thiazol-2-yl)benzyl]aminomethyl} pyridin-2-ylamino)-N-undecylacetamide,
   N,N-dimethyl-2-(6- {(pyridin-2-ylsulfonyl) [4-(thiazol-2-yl)benzyl] aminomethyl}-pyridin-2-ylamino)acetamide,
   (2-{2-[2-(benzyloxy)ethoxy]ethoxy} ethyl) (6-{(pyridin-2-ylsulfbnyl)[4-(thiazol-2-yl)-benzyl]aminomethyl}pyridin-2-ylamino)acetate,
   {2-[2-(2-hydroxyethoxy)ethoxy]ethyl} (6-{(pyridin-2-ylsulfonyl)[4-(thiazol-2-yl)-benzyl] aminomethyl}pyridin-2-ylamino)acetate,
   [(5-methyl-2-oxo-1,3-dioxolen-4-yl)methyl] (6-{(pyridin-2-ylsulfonyl)[4-(thiazol-2-yl)benzyl]aminomethyl}pyridin-2-ylamino)acetate,
   pivaloyloxymethyl (6-{(pyridin-2-ylsulfonyl)[4-(thiazol-2-yl)benzyl]aminomethyl}-pyridin-2-ylamino)acetate,
   [2-(dimethylamino)-2-oxoethyl] (6-{(pyridin-2-ylsulfonyl)[4-(thiazol-2-yl)benzyl]-aminomethyl}pyridin-2-ylamino)acetate,
   [2-(morpholin-4-yl)ethyl] (6-{(pyridin-2-ylsulfonyl)[4-(thiazol-2-yl)benzyl]aminomethyl}pyridin-2-ylamino)acetate,
   (2,2,2-trifluoroethyl) (6-{[4-(pyrazol-1-yl)benzyl](pyridin-3-ylsulfonyl)aminomethyl}-pyridin-2-ylamino)acetate,
   docosyl (6-{(pyridin-2-ylsulfonyl)[4-(thiazol-2-yl)benzyl]aminomethyl}pyridin-2-ylamino)acetate, or
   undecyl (6-{(pyridin-2-ylsulfonyl)[4-(thiazol-2-yl)benzyl]aminomethyl}pyridin-2-ylamino)acetate, etc.

Further, the present invention also provides:
(20) a pharmaceutical composition containing as an active ingredient the above-mentioned compound represented by the formula (I), the substituted carbonyl compound according to any one of (1) to (19) or a pharmaceutically acceptable salt thereof, and
(21) a pharmaceutical composition according to (20) for the prevention or treatment of respiratory diseases.

Representative preparation method of the compound of the present invention is shown below. With regard to the respective specific preparation method of the compounds of the present invention, they are explained in detail in the below-mentioned Examples.

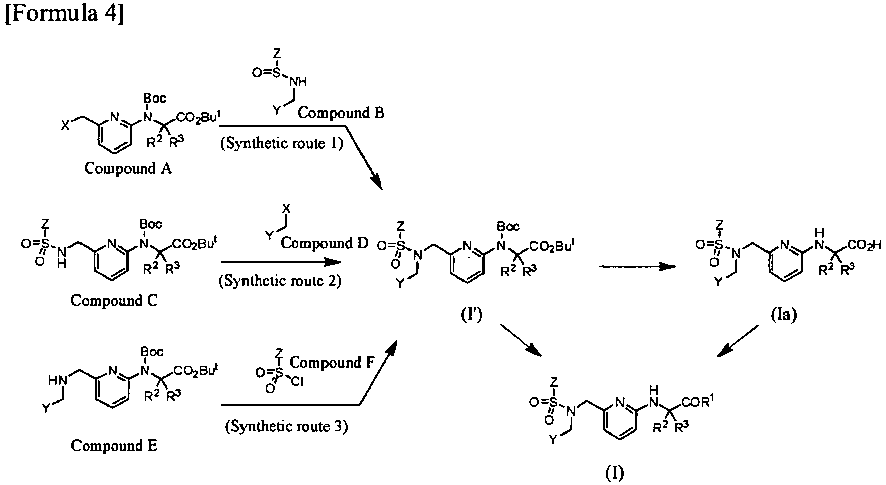

[wherein R¹, R², R³, Y and Z have the same meanings as defined above, X represents a hydroxy group, chloro group, bromo group, iodo group, methanesulfonyloxy group, benzenesulfonyloxy group, p-toluenesulfonyloxy group or trifluoromethanesulfonyloxy group, Boc represents a tert-butoxycarbonyl group, and Bu^{t} represents a tert-butyl group.]

Synthetic routes 1 to 3: Compound A and Compound B, or, Compound C and Compound D, or, Compound E and Compound F are each reacted in an organic solvent, in the presence of a condensing agent or a base, respectively, to prepare Compound (I') which is a precursor of the compound of the present invention. Compound (I) of the present invention can be directly derived from the precursor Compound (I'). Also, Compound (I) of the present invention can be derived from the active form (Ia) obtained by deprotecting the Boc group and Bu^{t} group of the precursor Compound (I') by an acid treatment.
The substituent(s) on the substituent Y and/or the substituent Z may be previously introduced before the preparation, or, after preparing a basic skeleton according to the above-mentioned process, a desired substituent(s) may be introduced into the basic skeleton by using the conventionally used synthetic method using an oxidation, reduction, alkylation, esterification, amidation, dehydration, deprotection, acetylation, hydrolysis, coupling reaction, cyclization and/or an optional combination thereof.
The preparation method of the synthetic intermediates of the compound according to the present invention will be mentioned in the following Examples in detail.

The objective compounds formed in each of the respective reactions can be obtained from a reaction mixture in accordance with the conventional methods. For example, after suitably neutralizing the reaction mixture, or removing insolubles by filtration in the case such insolubles are present, an organic solvent such as ethyl acetate that is not miscible with water is added followed by rinsing with water, separating the organic layer containing the objective compound, drying with a drying agent such as anhydrous magnesium sulfate and anhydrous sodium sulfate, and distilling off the solvent to obtain the objective compound.
The resulting objective compound can be separated and purified as necessary by suitably combining the conventional methods, examples of which include recrystallization; reprecipitation; or a method commonly used to separate and purify ordinary organic compounds (such as adsorption column chromatography method using a carrier such as silica gel and alkylated silica gel; ion exchange chromatography method; or normal or reverse phase column chromatography method using silica gel or alkylated silica gel (and preferably, high-performance liquid chromatography)).

Although the compound represented by the formula (I) of the present invention can be converted into a pharmaceutically acceptable salt in accordance with ordinary methods as necessary, it can also be separated directly from the reaction mixture as a salt.

In the case of using the compound represented by the formula (I), or a pharmaceutically acceptable salt thereof of the present invention, as a pharmaceutical, the compound, or pharmaceutically acceptable salt thereof, per se can be administered (as a bulk powder), or can be administered orally or parenterally (such as intravenous administration, intramuscular administration, intraperitoneal administration, transcutaneous administration, transtracheal administration, intracutaneous administration and subcutaneous administration) in a form such as a tablet, capsule, powder, syrup, granule, fine particles, pill, suspension, emulsion, transdermal preparation, suppository, ointment, lotion, inhalant and injection, which is prepared by mixing with a suitable pharmaceutically acceptable vehicle or diluent and the like.
These preparations are prepared by commonly known methods using additives such as vehicles, lubricants, binders, disintegrators, emulsifiers, stabilizers, corrigents or diluents and the like.

Examples of vehicles include organic vehicles and inorganic vehicles. Examples of organic vehicles include sugar derivatives such as lactose, sucrose, glucose, mannitol and sorbitol; starch derivatives such as cornstarch, potato starch, α-starch and dextrin; cellulose derivatives such as crystalline cellulose; gum Arabic; dextran; and pullulan. Examples of inorganic vehicles include light silicic acid anhydride; and sulfates such as calcium sulfate.

Examples of lubricants include stearic acid; stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; D,L-leucine; sodium lauryl sulfate; silicic acids such as silicic acid anhydride and silicic acid hydrate; and the above-mentioned starch derivatives listed as examples of the vehicles.

Examples of binders include hydroxypropyl cellulose, hydroxypropyl methylcellulose, polyvinyl pyrrolidone, Macrogol and the above-mentioned compounds listed as examples of the vehicles.

Examples of disintegrators include cellulose derivatives such as low substitution-degree hydroxypropyl cellulose, carboxymethyl cellulose, calcium carboxymethyl cellulose and internally-crosslinked calcium carboxymethyl cellulose; crosslinked polyvinyl pyrrolidone; and chemically modified starch or cellulose derivatives such as carboxymethyl starch and sodium carboxymethyl starch.

Examples of emulsifiers include colloidal clays such as bentonite and bee gum; anionic surfactants such as sodium lauryl sulfate; cationic surfactants such as benzalkonium chloride; and nonionic surfactants such as polyoxyethylene alkyl ethers, polyoxyethylene sorbitan fatty acid esters and sucrose fatty acid esters.

Examples of stabilizers include para-hydroxybenzoic acid esters such as methylparaben and propylparaben; alcohols such as chlorobutanol, benzyl alcohol and phenylethyl alcohol; benzalkonium chloride; phenols such as phenol and cresol; thimerosal; acetic anhydride; and sorbic acid.

Examples of corrigents include sweeteners such as sodium saccharin and aspartame; sour flavorings such as citric acid, malic acid and tartaric acid; and flavorings such as menthol, lemon extract and orange extract.

Examples of diluents include compounds ordinarily used as diluents, such as lactose, mannitol, glucose, sucrose, calcium sulfate, hydroxypropyl cellulose, microcrystalline cellulose, water, ethanol, polyethylene glycol, propylene glycol, glycerol, starch, polyvinyl pyrrolidone and mixtures thereof

Although the dosage of a compound represented by the formula (I) or a pharmaceutically acceptable salt thereof of the present invention can be varied according to conditions such as patient symptoms, age or body weight, the adult dosage per administration in the case of oral administration has a lower limit of 0.001 mg/Kg (preferably 0.01 mg/Kg) and an upper limit of 20 mg/Kg (preferably 10 mg/Kg), while the adult dosage per administration in the case of parenteral administration has a lower limit of 0.0001 mg/Kg (preferably 0.0005 mg/Kg) and an upper limit of 10 mg/kg (preferably 5 mg/Kg), administered corresponding to symptoms from 1 to 6 times per day.

### EXAMPLES

In the following, the present invention is explained in more detail by referring to Examples, Reference examples and Test examples, but the scope of the present invention is not limited to these ranges. Incidentally, the Rf value in Examples is a value measured by using a thin-layer chromatography (available from Merck, TLC plate silica gel 60F₂₅₄ (Trade name)), and the description in the parentheses represents an eluent(s) (volume ratio).

### [Example 1]

### (5-Phenylpentyl) (6-{(pyridin-2-ylsulfonyl)[4-(thiazol-2-yl)benzyl]aminomethyl}-pyridin-2-ylamino)acetate

To 1.1 ml of an N,N-dimethylformamide solution containing 174 mg (0.351 mmol) of (6-{(pyridin-2-ylsulfonyl)[4-(thiazol-2-yl)benzyl] amino methyl} pyridin-2-ylamino)acetic acid obtained in the same manner as in Reference example 3-(b) was added 0.2 ml of an N,N-dimethylformamide solution containing 97 mg (0.70 mmol) of potassium carbonate and 110 mg (0.454 mmol) of (5-phenylpentyl) methanesulfonate (see US6297257B), and the mixture was stirred at 45°C for 3 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate=1:1 (V/V)), and the fractions containing the objective material were concentrated under reduced pressure to obtain 170 mg of the title compound as white oil. (Yield: 75%)
Mass spectrum (FAB, m/z): 642 (M⁺+1).
¹H-NMR spectrum (DMSO-d₆, δ ppm): 8.63 (ddd, J=4.7, 1.7, 0.9Hz, 1H), 7.95 (ddd, J=7.8, 7.8, 1.7 Hz, 1H), 7.91 (d, J=3.3 Hz, 1H), 7.88-7.82 (m, 2H), 7.80 (ddd, J=7.8, 1.0, 0.9 Hz, 1H), 7.78 (d, J=3.3 Hz, 1H), 7.57 (ddd, J=7.8, 4.7, 1.0 Hz, 1H), 7.37-7.31 (m, 2H), 7.25-7.18 (m, 3H), 7.16-7.05 (m, 3H), 6.91 (t, J=6.0 Hz, 0.9H), 6.35 (d, J=8.3 Hz, 1H), 6.31 (d, J=6.8 Hz, 1H), 4.71 (s, 2H), 4.24 (s, 2H), 3.98 (t, J=6.6 Hz, 2H), 3.88 (d, J=6.0 Hz, 2H), 2.43 (t, J=7.7 Hz, 2H), 1.56-1.36 (m, 4H), 1.25-1.11 (m, 2H). Rf value: 0.25 (n-hexane:ethyl acetate=1:1).

### [Example 2]

### (10-Phenyldecyl) (6-{(pyridin-2-ylsulfonyl)[4-(thiazol-2-yl)benzyl)aminomethyl}-pyridin-2-ylamino)acetate hydrochloride

To 120 mg (0.242 mmol) of (6-{(pyridin-2-ylsulfonyl)[4-(thiazol-2-yl)benzyl]-aminomethyl}pyridin-2-ylamino)acetic acid obtained by the same manner as in Reference example 3-(b) were added 1.13 g (4.82 mmol) of 10-phenyl-1-decanol and 3 ml (12 mmol) of 4N hydrogen chloride/1,4-dioxane solution, and the mixture was stirred at room temperature for 17 hours, at 40°C for 8 hours, and further at room temperature for 15 hours. After completion of the reaction, the reaction mixture was concentrated under reduced pressure, a saturated aqueous sodium hydrogen carbonate solution was added to the residue, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate= 2:1→1:1 (V/V)), the fractions containing the objective material were concentrated under reduced pressure. The obtained residue was dissolved in 2 ml of diethyl ether, 1 ml of a diethyl ether solution saturated with hydrogen chloride was added thereto, and the mixture was concentrated under reduced pressure to obtain 120 mg of the title compound as white solid. (Yield: 63% as dihydrochloride)
Mass spectrum (FAB, m/z): 712 (M⁺+1).
¹H-NMR spectrum (DMSO-d₆, δ ppm): 8.73 (d, J=4.4 Hz, 1H), 8.06 (dd, J=7.4, 7.4 Hz, 1H), 7.94 (d, J=7.4 Hz, 1H), 7.90 (d, J=3.1 Hz, 1H), 7.82-7.78 (m, 2H), 7.78 (d, J=3.1 Hz, 1H), 7.68 (dd, J=7.4, 4.4 Hz, 1H), 7.58 (brs, 1H), 7.35-7.31 (m, 2H), 7.28-7.24 (m, 2H), 7.18-7.15 (m, 3H), 6.70 (brs, 1H), 6.58 (brs, 1H), 4.63 (s, 2H), 4.59 (s, 2H), 4.11 (s, 2H), 4.05 (t, J=6.6 Hz, 2H), 2.54 (t, J=7.7 Hz, 2H), 1.56-1.48 (m, 4H), 1.28-1.13 (m, 12H).

### [Example 3]

### 2-(6-{(Pyridin-2-ylsulfonyl)[4-(thiazol-2-yl)benzyl]aminomethyl}pyridin-2-ylamino)-N-undecylacetamide

To 1.5 ml of a methylene chloride solution containing 150 mg (0.303 mmol) of (6-{(pyridin-2-ylsulfonyl)[4-(thiazol-2-yl)benzyl]aminomethyl}pyridin-2-ylamino)-acetic acid obtained by the same manner as in Reference example 3-(b) was added 68 µl (0.32 mmol) of undecan-1-amine, then, 1.5 ml of a methylene chloride solution containing 61 mg (0.32 mmol) of 1-ethyl-3-(3-dimethylaminopropyl)carbodiimide hydrochloride and 44 µl (0.32 mmol) of triethylamine was added dropwise to the mixture over 3 minutes, further 24 mg (0.063 mmol) of O-benzotriazolyl-N,N,N',N'-tetramethyluronium hexafluorophosphoate was added to the mixture, and the mixture was stirred at room temperature for 15 hours. After completion of the reaction, water was added to the reaction mixture, and the mixture was extracted with methylene chloride. The organic layer was washed with a saturated sodium chloride solution, dried over anhydrous magnesium sulfate and concentrated under reduced pressure. The residue was applied to silica gel column chromatography (eluent; n-hexane:ethyl acetate=1:0→7:1 (V/V)), and the fractions containing the objective material were concentrated under reduced pressure to obtain 161 mg of the title compound as orange oil. (Yield: 82%)
Mass spectrum (CI, m/z): 649 (M⁺+1).
¹H-NMR spectrum (DMSO-d₆, δ ppm): 8.64 (ddd, J=4.7, 1.7, 0.8 Hz, 1H), 7.95 (ddd, J=7.7, 7.7, 1.7 Hz, 1H), 7.91 (d, J=3.3 Hz, 1H), 7.88-7.82 (m, 2H), 7.82-7.78 (m, 1H), 7.78 (d, J=3.3 Hz, 1H), 7.66 (t, J=5.7 Hz, 1H), 7.58 (ddd, J=7.7, 4.7, 1.0 Hz, 1H), 7.42-7.34 (m, 2H), 7.20 (dd, J=8.4, 7.2 Hz, 1H), 6.66 (t, J=5.9 Hz, 0.9H), 6.33 (d, J=8.4 Hz, 1H), 6.30 (d, J=7.2 Hz, 1H), 4.76 (s, 2H), 4.24 (s, 2H), 3.70 (d, J=5.9 Hz, 2H), 3.03(q, J=6.4 Hz, 2H), 1.41-1.02 (m, 18H), 0.83 (t, J=6.8 Hz, 3H).
Rf value: 0.36 (ethyl acetate:methanol=50:1).

### [Example 4]

### N,N-Dimethyl-2-(6-{(pyridin-2-ylsulfonyl)[4-(thiazol-2-yl)benzyl]aminomethyl}-pyridin-2-ylamino)acetamide

To 1.5 ml of an N,N-dimethylformamide solution containing 150 mg (0.303 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) were added 0.16 ml (0.32 mmol) of 2.0M dimethylamine/tetrahydrofuran solution, 122 mg (0.32 mmol) of O-benzotriazolyl-N,N,N',N'-tetramethyluronium hexafluorophosphate and 44 mg (0.33 mmol) of 1-hydroxybenzotriazole, and the mixture was stirred at room temperature for 15 hours. After completion of the reaction, a saturated aqueous sodium hydrogen carbonate solution was added to the reaction mixture, and the mixture was extracted with toluene. The organic layer was washed with a saturated sodium chloride solution, dried over anhydrous magnesium sulfate and concentrated under reduced pressure. The residue was applied to silica gel column chromatography (eluent; ethyl acetate), and the fractions containing the objective material were concentrated under reduced pressure to obtain 96 mg of the title compound as pale yellow foam. (Yield: 61 %)
Mass spectrum (CI, m/z): 523 (M⁺+1).
¹H-NMR spectrum (DMSO-d₆, δ ppm): 8.66 (ddd, J=4.6, 1.8, 0.9 Hz, 1H), 7.98 (ddd, J=7.8, 7.8, 1.8 Hz, 1H), 7.92 (d, J=3.2 Hz, 1H), 7.88-7.82 (m, 3H), 7.78 (d, J=3.2 Hz, 1H), 7.60 (ddd, J=7.8, 4.6, 1.2 Hz, 1H), 7.37-7.31 (m, 2H), 7.20 (dd, J=8.1, 7.1 Hz, 1H), 6.41 (d, J=8.1 Hz, 1H), 6.37 (t, J=5.1 Hz, 0.9H), 6.28 (d, J=7.1 Hz, 1H), 4.71 (s, 2H), 4.28 (s, 2H), 3.95 (d, J=5.1 Hz, 2H), 2.99 (s, 3H), 2.84 (s, 3H).
Rf value: 0.25 (ethyl acetate:methanol=50:1).

### [Example 5]

### (2-{2-[2-(Benzyloxy)ethoxy]ethoxy}ethyl)(6-{(pyridin-2-ylsulfonyl)[4-(thiazol-2-yl)-benzyl]aminomethyl}pyridin-2-ylamino)acetic acid

To 1 ml of an N,N-dimethylformamide solution containing 200 mg (0.404 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) were added 89 mg (0.64 mmol) of potassium carbonate and 194 mg (0.609 mmol) of (2-{2-[2-(benzyloxy)ethoxy]ethoxy}ethyl) methanesulfonate (see US2008/207505A), and the mixture was stirred at 50°C for 7.5 hours, and then, at room temperature for 15 hours. After completion of the reaction, water was added to the reaction mixture, and the mixture was extracted with ethyl acetate. The organic layer was washed with a saturated aqueous sodium chloride solution, dried over anhydrous sodium sulfate and concentrated under reduced pressure. The residue was applied to silica gel column chromatography (eluent; n-hexane:ethyl acetate=1:1→1:50 (V/V)), and the fractions containing the objective material were concentrated under reduced pressure to obtain 287 mg of the title compound as colorless oil. (Yield: 99%)
Mass spectrum (FAB, m/z): 718 (M⁺+1).
¹H-NMR spectrum (DMSO-d₆, δ ppm): 8.65 (ddd, J=4.7, 1.8, 0.9 Hz, 1H), 7.96 (ddd, J=7.7, 7.7, 1.8 Hz, 1H), 7.91 (d, J=3.1 Hz, 1H), 7.88-7.80 (m, 3H), 7.77 (d, J=3.1 Hz, 1H), 7.58 (ddd, J=7.7, 4.7, 1.2 Hz, 1H), 7.37-7.23 (m, 7H), 7.20 (dd, J=8.1, 7.0 Hz, 1H), 6.89 (t, J=6.1 Hz, 0.9H), 6.35 (d, J=8.1 Hz, 1H), 6.30 (d, J=7.0 Hz, 1H), 4.70 (s, 2H), 4.45 (s, 2H), 4.25 (s, 2H), 4.16-4.10 (m, 2H), 3.89 (d, J=6.1 Hz, 2H), 3.59-3.53 (m, 2H), 3.51 (s, 4H), 3.45 (s, 4H).
Rf value: 0.59 (ethyl acetate:methanol=50:1).

### [Example 6]

### {2-[2-(2-Hydroxyethoxy)ethoxy]ethyl}(6-{(pyridin-2-ylsulfonyl)[4-(thiazol-2-yl)-benzyl]aminomethyl}pyridin-2-ylamino)acetate

To 189 mg (0.263 mmol) of (2-{2-[2-(benzyloxy)ethoxy]ethoxy}ethyl) (6-{(pyridin-2-ylsulfonyl)[4-(thiazol-2-yl)benzyl]aminomethyl}pyridin-2-ylamino)acetate obtained in Example 5 was added 5.3 ml of trifluoroacetic acid, and the mixture was stirred at 60°C for 12 hours. The reaction mixture was concentrated under reduced pressure, 10 ml of a saturated aqueous sodium hydrogen carbonate solution was added to the residue, and the mixture was extracted with ethyl acetate. After the organic layer was dried over anhydrous sodium sulfate, the mixture was concentrated under reduced pressure. To the residue were added 5 ml of acetonitrile and 1 ml of water, and the mixture was stirred at room temperature for 22 hours. After completion of the reaction, 3 ml of a saturated aqueous sodium hydrogen carbonate solution was added to the reaction mixture, and the mixture was extracted with ethyl acetate. The organic layer was washed with a saturated aqueous sodium chloride solution, dried over anhydrous sodium sulfate and concentrated under reduced pressure. The residue was applied to silica gel column chromatography (eluent; ethyl acetate:methanol=1:0→10:1 (V/V)), and the fractions containing the objective material were concentrated under reduced pressure to obtain 148 mg of the title compound as colorless oil. (Yield: 90%)
Mass spectrum (FAB, m/z): 628 (M⁺+1).
¹H-NMR spectrum (DMSO-d₆, δ ppm): 8.66 (ddd, J=4.7, 1.6, 0.8 Hz, 1H), 7.97 (ddd, J=7.7, 7.7, 1.6 Hz, 1H), 7.92 (d, J=3.3 Hz, 1H), 7.89-7.80 (m, 3H), 7.78 (d, J=3.3 Hz, 1H), 7.60 (ddd, J=7.7, 4.7, 1.2 Hz, 1H), 7.37-7.30 (m, 2H), 7.21 (dd, J=8.3, 7.2 Hz, 1H), 6.89 (t, J=6.2 Hz, 0.9H), 6.35 (d, J=8.3 Hz, 1H), 6.31 (d, J=7.2 Hz, 1H), 4.70 (s, 2H), 4.55 (t, J=5.4 Hz, 1H), 4.25 (s, 2H), 4.17-4.10 (m, 2H), 3.89 (d, J=6.2 Hz, 2H), 3.59-3.52 (m, 2H), 3.49-3.42 (m, 6H), 3.40-3.34 (m, 2H).
Rf value: 0.18 (ethyl acetate:methanol=50:1).

### [Example 7]

### [(5-Methyl-2-oxo-1,3-dioxolen-4-yl)methyl](6-{(pyridin-2-ylsulfonyl)[4-(thiazol-2-yl)benzyl]aminomethyl}pyridin-2-ylamino)acetate

To 1.2 ml of an N,N-dimethylformamide solution containing 198 mg (0.400 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) was added 0.2 ml of an N,N-dimethylformamide solution containing 71 mg (0.51 mmol) of potassium carbonate and 89 mg (0.60 mmol) of 4-chloromethyl-5-methyl-1,3-dioxolen-2-one, and the mixture was stirred at room temperature for 15 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate=1:2 (V/V)), and the fractions containing the objective material were concentrated under reduced pressure to obtain 203 mg of the title compound as pale yellowish-brown foam. (Yield: 84%)
Mass spectrum (FAB, m/z): 608 (M⁺+1).
¹H-NMR spectrum (DMSO-d₆, δ ppm): 8.66 (ddd, J=4.7, 1.8, 0.8 Hz, 1H), 7.98 (ddd, J=7.7, 7.7, 1.8 Hz, 1H), 7.91 (d, J=3.3 Hz, 1H), 7.87-7.81 (m, 3H), 7.78 (d, J=3.3 Hz, 1H), 7.60 (ddd, J=7.7, 4.7, 1.1 Hz, 1H), 7.36-7.29 (m, 2H), 7.21 (dd, J=8.3, 7.2 Hz, 1H), 6.95 (t, J=6.2 Hz, 0.8H), 6.36 (d, J=8.3 Hz, 1H), 6.31 (d, J=7.2 Hz, 1H), 4.95 (s, 2H), 4.69 (s, 2H), 4.24 (s, 2H), 3.95 (d, J=6.2 Hz, 2H), 2.07 (s, 3H).
Rf value: 0.67 (ethyl acetate:methanol=50:1).

### [Example 8]

### Pivaloyloxymethyl (6-{(pyridin-2-ylsulfonyl)[4-(thiazol-2-yl)benzyl)aminomethyl}-pyridin-2-ylamino)acetate

To 1.2 ml of an N,N-dimethylformamide solution containing 174 mg (0.351 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) was added 0.2 ml of an N,N-dimethylformamide solution containing 62 mg (0.45 mmol) of potassium carbonate and 79 mg (0.52 mmol) of chloromethyl pivalate, and the mixture was stirred at room temperature for 15 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate=1:1 (V/V)), and the fractions containing the objective material were concentrated under reduced pressure to obtain 141 mg of the title compound as white foam. (Yield: 66%) Mass spectrum (FAB, m/z): 610 (M⁺+1).
¹H-NMR spectrum (DMSO-d₆, δ ppm): 8.66 (ddd, J=4.7, 1.8, 0.8 Hz, 1H), 7.98 (ddd, J=7.7, 7.7, 1.8 Hz, 1H), 7.91 (d, J=3.3 Hz, 1H), 7.88-7.80 (m, 3H), 7.78 (d, J=3.3 Hz, 1H), 7.60 (ddd, J=7.7, 4.7, 1.1 Hz, 1H), 7.36-7.31 (m, 2H), 7.21 (dd, J=8.2, 7.1 Hz, 1H), 6.97 (t, J=6.1 Hz, 0.8H), 6.35 (d, J=8.2 Hz, 1H), 6.33 (d, J=7.1 Hz, 1H), 5.71 (s, 2H), 4.71 (s, 2H), 4.25 (s, 2H), 3.95 (d, J=6.1 Hz, 2H), 1.04 (s, 9H).
Rf value: 0.22 (n-hexane:ethyl acetate=1:1).

### [Example 9]

### [2-(Dimethylamino)-2-oxoethyl] (6-{(pyridin-2-ylsulfonyl)[4-(thiazol-2-yl)benzyl]-aminomethyl}pyridin-2-ylamino)acetate

To 1 ml of an N,N-dimethylformamide solution containing 174 mg (0.351 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) were added 97 mg (0.70 mmol) of potassium carbonate and 47 µl (0.46 mmol) of2-chloro-N,N-dimethylacetamide, and the mixture was stirred at room temperature for 4 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; ethyl acetate:methanol=9:1 (V/V)), and the fractions containing the objective material were concentrated under reduced pressure to obtain 203 mg of the title compound as white foam substantially quantitatively.
Mass spectrum (FAB, m/z): 581 (M⁺+1).
¹H-NMR spectrum (DMSO-d₆, δ ppm): 8.67 (ddd, J=4.7, 1.8, 0.9 Hz, 1H), 7.96 (ddd, J=7.7, 7.7, 1.8 Hz, 1H), 7.92 (d, J=3.2 Hz, 1H), 7.88-7.83 (m, 2H), 7.81 (ddd, J=7.8, 1.0, 0.9 Hz, 1H), 7.78 (d, J=3.2 Hz, 1H), 7.59 (ddd, J=7.7, 4.7, 1.0 Hz, 1H), 7.38-7.33 (m, 2H), 7.21 (dd, J=8.3, 7.1 Hz, 1H), 6.92 (t, J=6.0 Hz, 0.8H), 6.35 (d, J=8.3 Hz, 1H), 6.31 (d, J=7.1 Hz, 1H), 4.77 (s, 2H), 4.73 (s, 2H), 4.27 (s, 2H), 3.98 (d, J=6.0 Hz, 2H), 2.87 (s, 3H), 2.78 (s, 3H).
Rf value: 0.20 (ethyl acetate:methanol=50:1).

### [Example 10]

### [2-(Morpholin-4-yl)ethyl](6-{(pyridin-2-ylsulfonyl)[4-(thiazol-2-yl)benzyl]aminomethyl pyridin-2-ylamino)acetate

To 1.3 ml of an N,N-dimethylformamide solution containing 174 mg (0.351 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) were added 138 mg (1.00 mmol) ofpotassium carbonate and 210 mg (1.00 mmol) of [2-(morpholin-4-yl)ethyl]methanesulfonate (see The Journal of Medicinal Chemistry, 51, 1904 (2008)), and the mixture was stirred at 45°C for 4 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; ethyl acetate: methanol=15:1→10:1 (V/V)), and the fractions containing the objective material were concentrated under reduced pressure to obtain 36 mg of the title compound as pale yellow foam. (Yield: 17%)
Mass spectrum (FAB, m/z): 609 (M⁺+1).
¹H-NMR spectrum (DMSO-d₆, δ ppm): 8.66 (ddd, J=4.7, 1.7, 0.8 Hz, 1H), 7.98 (ddd, J=7.7, 7.7, 1.7 Hz, 1H), 7.92 (d, J=3.3 Hz, 1H), 7.88-7.81 (m, 3H), 7.78 (d, J=3.3 Hz, 1H), 7.60 (ddd, J=7.6, 4.7, 1.1 Hz, 1H), 7.37-7.31 (m, 2H), 7.22 (dd, J=8.2, 7.2 Hz, 1H), 6.93 (t, J=6.1 Hz, 1H), 6.35 (d, J=8.2 Hz, 1H), 6.31 (d, J=7.2 Hz, 1H), 4.71 (s, 2H), 4.25 (s, 2H), 4.10 (t, J=5.9 Hz, 2H), 3.88 (d, J=6.1 Hz, 2H), 3.48-3.41 (m, 4H), 2.45 (t, J=5.9 Hz, 2H), 2.31-2.24 (m, 4H).

### [Example 11]

### (2,2,2-Trifluoroethyl)(6-{[4-(pyrazol-1-yl)benzyl](pyridin-3-ylsulfonyl)aminomethyl}-pyridin-2-ylamino)acetate

To 1.0 ml of a 2,2,2-trifluoroethanol solution containing 144 mg (0.300 mmol) of (6-{[4-(pyrazol-1-yl)benzyl](pyridin-3-ylsulfonyl)aminomethyl}pyridin-2-ylamino)-acetic acid obtained in Reference example 4-(c) was added 1.25 ml (5.0 mmol) of 4N hydrogen chloride/1,4-dioxane solution, and the mixture was stirred at 60°C for 24 hours. After completion of the reaction, the reaction mixture was concentrated under reduced pressure, a saturated aqueous sodium hydrogen carbonate solution was added to the residue, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate=1:2→1:3 (V/V)), and the fractions containing the objective material were concentrated under reduced pressure to obtain 45 mg of the title compound as white foam. (Yield: 27%)
Mass spectrum (FAB, m/z): 561 (M⁺+1).
¹H-NMR spectrum (CDCl₃, δ ppm): 9.09 (d, J=1.5 Hz, 1H), 8.90 (d, J=4.4 Hz, 1H), 9.21-8.17 (m, 1H), 7.89(d, J=2.5 Hz, 1H), 7.69 (d, J=1.7 Hz, 1H), 7.66 (t, J=6.1 Hz, 1H), 7.57 (dd, J=7.9, 4.8 Hz, 1H), 7.56-7.52 (m, 2H), 7.46-7.43 (m, 2H), 6.99 (d, J=7.6 Hz, 1H), 6.45 (dd, J=2.5, 1.7 Hz, 1H), 6.32 (d, J=8.3 Hz, 1H), 4.55 (s, 2H), 4.54 (s, 2H), 4.51 (q, J=8.2 Hz, 2H), 4.00 (d, J=4.8 Hz, 2H).
Rfvalue: 0.10 (n-hexane:ethyl acetate=1:1).

### [Example 12]

### Docosyl (6-{(pyridin-2-ylsulfonyl)[4-(thiazol-2-yl)benzyl]aminomethyl}pyridin-2-yl-amino)acetate

To 1.3 ml of an N,N-dimethylformamide solution containing 174 mg (0.351 mmol) of (6-{(pyridin-2-ylsulfonyl)[4-(thiazol-2-yl)benzyl]aminomethyl}pyridin-2-ylamino)acetic acid obtained by the same manner as in Reference example 3-(b) were added 73 mg (0.53 mmol) of potassium carbonate and 178 mg (0.440 mmol) of docosyl methanesulfonate (see Journal of Medicinal Chemistry, 50, 1645 (2007)), and the mixture was stirred at 50°C for 5 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate= 2:1→1:1 (V/V)), and the fractions containing the objective material were concentrated under reduced pressure to obtain 141 mg of the title compound as white foam. (Yield: 50%)
Mass spectrum (FAB, m/z): 804 (M⁺+1).
¹H-NMR spectrum (DMSO-d₆, δ ppm): 8.64 (ddd, J=4.7, 1.7, 0.8 Hz, 1H), 7.95 (ddd, J=7.7, 7.7, 1.7 Hz, 1H), 7.91 (d, J=3.2 Hz, 1H), 7.89-7.82 (m, 2H), 7.81-7.76 (m, 2H), 7.58 (ddd, J=7.7, 4.7, 1.0 Hz, 1H), 7.37-7.32 (m, 2H), 7.20 (dd, J=8.2, 7.1 Hz, 1H), 6.91 (t, J=6.1 Hz, 0.9H), 6.35 (d, J=8.2 Hz, 1H), 6.30 (d, J=7.1 Hz, 1H), 4.73 (s, 2H), 4.23 (s, 2H), 3.98 (t, J=6.6 Hz, 2H), 3.88 (d, J=6.1 Hz, 2H), 1.31-1.03 (m, 40H), 0.88-0.81 (m, 3H).
Rf value: 0.47 (n-hexane:ethyl acetate=1:1).

### [Example 13]

### Undecyl (6-{(pyridin-2-ylsulfonyl)[4-(thiazol-2-yl)benzyl]aminomethyl}pyridin-2-yl-amino)acetate hydrochloride

To 110 mg (0.222 mmol) of (6-{(pyridin-2-ylsulfonyl)[4-(thiazol-2-yl)benzyl]-aminomethyl}pyridin-2-ylamino)acetic acid obtained by the same manner as in Reference example 3-(b) were added 0.83 ml (3.3 mmol) of 4N hydrogen chloride/1,4-dioxane solution and 0.83 ml (4.0 mmol) of 1-undecanol, and the mixture was stirred at room temperature for 15 hours. After completion of the reaction, the reaction mixture was concentrated under reduced pressure, a saturated aqueous sodium hydrogen carbonate solution was added to the residue, and the mixture was extracted with ethyl acetate. The organic layer was washed with a saturated sodium chloride solution, dried over anhydrous magnesium sulfate and concentrated under reduced pressure. The residue was applied to silica gel column chromatography (eluent; n-hexane:ethyl acetate=3:1→1:1 (V/V)), the fractions containing the objective material were concentrated under reduced pressure. The obtained residue was dissolved in 5 ml of diethyl ether, 0.5 ml of a diethyl ether solution saturated with hydrogen chloride was added to the mixture, and the mixture was concentrated under reduced pressure to obtain 118 mg of the title compound as white solid. (Yield: 74% as dihydrochloride)
Mass spectrum (FAB, m/z): 650 (M⁺+1).
¹H-NMR spectrum (DMSO-d₆, δ ppm): 8.71 (d, J=4.4 Hz, 1H), 8.04 (dd, J=6.7, 6.7 Hz, 1H), 7.94-7.89 (m, 2H), 7.84-7.79 (m, 2H), 7.79 (d, J=3.1 Hz, 1H), 7.67 (dd, J=6.7, 4.4 Hz, 1H), 7.50 (brs, 1H), 7.36-7.31 (m, 2H), 6.62 (brs, 1H), 6.52 (brs, 1H), 4.65 (s, 2H), 4.51 (brs, 2H), 4.14-3.97 (m, 4H), 1.55-1.46 (m, 2H), 1.29-1.06 (m, 16H), 0.84 (t, J=7.0 Hz, 3H).

The compounds used in Examples were synthesized as follows.

### [Reference example 1]

### tert-Butyl [(6-aminomethylpyridin-2-yl)tert-butoxycarbonylamino]acetate

### 1-(a) tert-Butyl[tert-butoxycarbonyl(6-ethoxycarbonylpyridin-2-yl)amino]acetate

To 362 ml of an N,N-dimethylformamide solution containing 15.7 g (0.360 mol) of sodium hydride (55% dispersed material in mineral oil) was added dropwise 300 ml of an N,N-dimethylformamide solution containing 81.2 g (0.305 mol) of ethyl 6-tert-butoxycarbonylaminopyridin-2-carboxylate (see WO2006/074884A) under argon atmosphere and under ice-cooling over 20 minutes, and the mixture was stirred at room temperature for 1 hour. Then, 54.0 ml (0.366 mol) of tert-butyl bromoacetate was added dropwise under ice-cooling over 10 minutes to the mixture, and the mixture was further stirred at room temperature for 1 hour. After completion of the reaction, to the reaction mixture was added an aqueous solution in which 1.77 g (33.0 mmol) of ammonium chloride had been dissolved in 300 ml of water, and the mixture was extracted with toluene. The organic layer was washed with a saturated sodium chloride solution, dried over anhydrous magnesium sulfate and concentrated under reduced pressure. The obtained residue was applied to silica gel column chromatography (eluent; n-hexane:ethyl acetate=9:1→4: (V/V)), and the fractions containing the objective material were concentrated under reduced pressure to obtain 108 g of the title compound as pale yellow oil. (Yield: 93%)
Mass spectrum (CI, m/z): 381 (M⁺+1).
¹H-NMR spectrum (CDCl₃, δ ppm): 8.04 (d, J=7.8 Hz, 1H), 7.81 (dd, J=7.6, 1.5 Hz, 1H), 7.76 (dd, J=7.8, 7.6 Hz, 1H), 4.67 (s, 2H), 4.40 (q, J=7.1 Hz, 2H), 1.52 (s, 9H), 1.45 (s, 9H), 1.40 (t, J=7.1 Hz, 3H).

### 1-(b) tert-Butyl [tert-butoxycarbonyl(6-hydroxymethylpyridin-2-yl)amino]acetate

To 195 ml of an ethanol solution containing 98.8 g (0.260 mol) of tert-butyl [tert-butoxycarbonyl(6-ethoxycarbonylpyridin-2-yl)amino] acetate obtained in Reference example 1-(a) was added dropwise 195 ml of an ethanol solution containing 34.6 g (0.312 mol) of calcium chloride under ice-cooling over 20 minutes. After completion of the dropwise addition, 105 ml (0.315 mol) of 3M sodium borohydride/- tetraethylene glycol dimethyl ether solution was added dropwise to the mixture at 35°C or lower over 20 minutes, and the mixture was further stirred at room temperature for 15 minutes. After completion of the reaction, the reaction mixture was added dropwise to 195 ml of an aqueous solution containing 17.8 ml of acetic acid in water under ice-cooling over 10 minutes, and the mixture was stirred at room temperature for 1 hour. Then, 315 ml of water was added to the mixture, and the mixture was extracted with toluene. The organic layer was successively washed with a saturated aqueous sodium hydrogen carbonate solution, water, and then, a saturated aqueous sodium chloride solution, and the mixture was concentrated under reduced pressure. The obtained residue was applied to silica gel column chromatography (eluent; n-hexane:ethyl acetate=4:1→3:2 (V/V)), and the fractions containing the objective material were concentrated under reduced pressure to obtain 81.1 g of the title compound as pale yellow oil. (Yield: 92%)
Mass spectrum (CI, m/z): 339 (M⁺+1).
¹H-NMR spectrum (CDCl₃, δ ppm): 7.74 (d, J=8.2 Hz, 1H), 7.63 (dd, J=8.2, 7.4 Hz, 1H), 6.93-6.98 (m, 1H), 4.68-4.65 (m, 2H), 4.54 (s, 2H), 3.39 (t, J=5.3 Hz, 1H), 1.54 (s, 9H), 1.46 (s, 9H).

### 1-(c) tert-Butyl [tert-butoxycarbonyl(6-formylpyridin-2-yl)amino]acetate

To 130 ml of a methylene chloride solution containing 12.9 g (30.4 mmol) of Dess-martin reagent were added dropwise 50 ml of a methylene chloride solution containing 10.0 g (29.6 mmol) of tert-butyl [tert-butoxycarbonyl(6-hydroxymethylpyridin-2-yl)amino]acetate obtained in Reference example 1-(b) under argon atmosphere and under ice-cooling over 20 minutes. After completion of the dropwise addition, the mixture was stirred at room temperature for 2 hours. After completion of the reaction, to the reaction mixture was added 305 ml of 0.1% aqueous sodium thiosulfate solution, and the mixture was extracted with methylene chloride. The organic layer was successively washed with 0.5N aqueous sodium hydroxide solution and a saturated aqueous sodium chloride solution, dried over anhydrous magnesium sulfate, and concentrated under reduced pressure to obtain 9.61 g of the title compound as pale yellow oil substantially quantitatively.
Mass spectrum (EI, m/z): 336 (M⁺).
¹H-NMR spectrum (DMSO-d₆, δ ppm): 9.82 (s, 1H), 8.11-7.99 (m, 2H), 7.68 (dd, J=6.6, 1.5 Hz, 1H), 4.58 (s, 2H), 1.48 (s, 9H), 1.42 (s, 9H).

### 1-(d) tert-Butyl [tert-butoxycarbonyl(6-hydroxyiminomethylpyridin-2-yl)amino]acetate

To 29 ml of a methanol solution containing 2.88 g (8.56 mmol) of tert-butyl [tert-butoxycarbonyl(6-formylpyridin-2-yl)amino] acetate obtained in Reference example 1-(c) were added 0.650 g (9.35 mmol) of hydroxyl ammonium chloride and 3.5 ml (43 mmol) of pyridine, and the mixture was stirred at room temperature for 1 hour. After completion of the reaction, the reaction mixture was concentrated under reduced pressure. Ethyl acetate was added to the obtained residue, and the mixture was successsively washed with a 5% aqueous potassium hydrogen sulfate solution, a saturated aqueous sodium hydrogen carbonate solution and a saturated aqueous sodium chloride solution, dried over anhydrous magnesium sulfate, and concentrated under reduced pressure. The obtained residue was applied to silica gel column chromatography (eluent; n-hexane:ethyl acetate=3:2 (V/V)), and the fractions containing the objective material were concentrated under reduced pressure to obtain 2.76 g of the title compound as colorless oil. (Yield: 92%)
Mass spectrum (EI, m/z): 351 (M⁺).
¹H-NMR spectrum (CDCl₃, δ ppm): 8.06 (s, 1H), 7.91 (s, 1H), 7.85 (d, J=8.2 Hz, 1H), 7.65 (dd, J=8.2, 7.6 Hz, 1H), 7.47 (dd, J=7.6, 0.7 Hz, 1H), 4.59 (s, 2H), 1.53 (s, 9H), 1.45 (s, 9H).

### 1-(e) tert-Butyl [(6-aminomethylpyridin-2-yl)tert-butoxycarbonylamino]acetate

To 49 ml of an ethanol solution containing 2.75 g (7.83 mmol) of tert-butyl [tert-butoxycarbonyl(6-hydroxyiminomethylpyridin-2-yl)amino] acetate obtained in Reference example 1-(d) was added 0.98 g of 10% palladium-active carbon (50% hydrate), and the mixture was stirred under 1 atm hydrogen atmosphere at room temperature for 1 hour. After completion of the reaction, insoluble materials were filtered off, and the filtrate was concentrated under reduced pressure to obtain 2.48 g of the title compound as colorless oil. (Yield: 94%)
Mass spectrum (CI, m/z): 338 (M⁺+1).
¹H-NMR spectrum (CDCl₃, δ ppm): 7.68 (d, J=8.3 Hz, 1H), 7.58 (dd, J=8.3, 7.4 Hz, 1H), 6.91 (d, J=7.4 Hz, 1H), 4.57 (s, 2H), 3.85 (s, 2H), 1.53 (s, 9H), 1.46 (s, 9H).

### [Reference example 2]

### N-[4-(thiazol-2-yl)benzyl]pyridin-2-ylsulfonamide

### 2-(a) 4-(Thiazol-2-yl)benzyl alcohol

To a mixed solution comprising 20 ml of ethanol and 0.46 ml of tetrahydrofuran containing 1.57 g (8.30 mmol) of 4-(thiazol-2-yl)benzaldehyde (see JP 2001-519414A) was added 157 mg (4.15 mmol) of sodium borohydride, and the mixture was stirred at room temperature for 1.5 hours. After completion of the reaction, water was added to the reaction mixture, and the mixture was extracted with ethyl acetate. The organic layer after separating the liquids was dried over anhydrous sodium sulfate and concentrated under reduced pressure. The obtained residue was applied to silica gel column chromatography (eluent; n-hexane:ethyl acetate=2:1→1:1 (V/V)), and the fractions containing the objective material were concentrated under reduced pressure to obtain 1.49 g of the title compound as white solid. (Yield: 94%)
Mass spectrum (CI, m/z): 192 (M⁺+1).
¹H-NMR spectrum (CDCl₃, δ ppm): 7.94-7.89 (m, 2H), 7.84 (d, J=3.2 Hz, 1H), 7.44-7.38 (m, 2H), 7.32 (d, J=3.2 Hz, 1H), 4.72 (d, J=5.9 Hz, 2H), 2.41 (t, J=5.9 Hz, 1H).

### 2-(b) 4-(Thiazol-2-yl)benzyl bromide

To 55.8 ml of a tetrahydrofuran solution containing 1.31 g (6.85 mmol) of 4-(thiazol-2-yl)benzyl alcohol obtained in 2-(a) were added 1.80 g (8.90 mmol) of triphenylphosphine and 1.59 g (8.93 mmol) of N-bromosuccinimide, and the mixture was stirred at room temperature for 1.5 hours. After completion of the reaction, a saturated aqueous sodium hydrogen carbonate solution was added to the reaction mixture, and the mixture was extracted with ethyl acetate. The organic layer after separating the liquids was washed with a saturated aqueous sodium chloride solution, dried over anhydrous sodium sulfate and concentrated under reduced pressure. The obtained residue was applied to silica gel column chromatography (eluent; n-hexane:ethyl acetate=2:1 (V/V)), and the fractions containing the objective material were concentrated under reduced pressure to obtain 1.26 g of the title compound as pale yellow solid. (Yield: 72%)
Mass spectrum (CI, m/z): 254 (M⁺+1).
¹H-NMR spectrum (CDCl₃, δ ppm): 7.98-7.92 (m, 2H), 7.88 (d, J=3.3 Hz, 1H), 7.50-7.45 (m, 2H), 7.35 (d, J=3.3 Hz, 1H), 4.52 (s, 2H).

### 2-(c) 2-{4-[Bis(tert-butoxycarbonyl)aminomethyl]phenyl}thiazole

To 16 ml of an N,N-dimethylformamide solution containing 1.25 g (4.92 mmol) of 4-(thiazol-2-yl)benzyl bromide obtained in Reference example 2-(b) were added 1.28 g (5.89 mmol) of di-tert-butyl iminodicarboxylate and 1.35 g (9.76 mmol) of potassium carbonate, and the mixture was stirred at room temperature for 3 hours. After completion of the reaction, water was added to the reaction mixture, and the mixture was extracted with ethyl acetate. The organic layer after separating the liquids was washed with a saturated aqueous sodium chloride solution, dried over anhydrous sodium sulfate and concentrated under reduced pressure. The obtained residue was applied to silica gel column chromatography (eluent; n-hexane:ethyl acetate=2:1 (V/V)), and the fractions containing the objective material were concentrated under reduced pressure to obtain 2.05 g of the title compound as colorless oil substantially quantitatively.
¹H-NMR spectrum (CDCl₃, δ ppm): 7.95-7.89 (m, 2H), 7.85 (d, J=3.4 Hz, 1H), 7.39-7.34 (m, 2H), 7.32 (d, J=3.4 Hz, 1H), 4.81 (s, 2H), 1.46 (s, 9H), 1.46 (s, 9H).

### 2-(d) 4-(Thiazol-2-yl)benzylamine hydrochloride

To 2 ml of a methylene chloride solution containing 1.91 g (4.89 mmol) of 2-{4-[bis(tert-butoxycarbonyl)aminomethyl]phenyl}thiazole obtained in Reference example 2-(c) was added 20 ml (80 mmol) of a 1,4-dioxane solution containing 4N hydrogen chloride, and the mixture was stirred at room temperature for 1 hour. After completion of the reaction, the reaction mixture was concentrated under reduced pressure to obtain 1.37 g of a crude product containing the title compound as white solid substantially quantitatively.
¹H-NMR spectrum (DMSO-d₆, δ ppm): 8.56 (brs, 2H), 8.03-7.97 (m, 2H), 7.95 (d, J=3.2 Hz, 1H), 7.83 (d, J=3.2 Hz, 1H), 7.67-7.60 (m, 2H), 4.12-4.03 (m, 2H).

### 2-(e) N-[4-(thiazol-2-yl)benzyl]pyridin-2-ylsulfonamide

To 4.3 ml of a methylene chloride solution containing 0.30 g (1.1 mmol) of 4-(thiazol-2-yl)benzylamine hydrochloride obtained in Reference example 2-(d) were added 0.87 ml (6.2 mmol) of triethylamine and 0.22 g (1.2 mmol) of 2-pyridylsulfonyl chloride (see The Journal of Organic Chemistry, 54, 389 (1989)), and the mixture was stirred at room temperature for 3 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The obtained residue was applied to silica gel column chromatography (eluent; n-hexane:ethyl acetate=1:4 (V/V)), and the fractions containing the objective material were concentrated under reduced pressure to obtain 284 mg of the title compound as white solid.
(Yield: 75%)
Mass spectrum (CI, m/z): 332 (M⁺+1).
¹H-NMR spectrum (CDCl₃, δ ppm): 8.66 (ddd, J=4.6, 1.7, 1.0 Hz, 1H), 7.98 (ddd, J=7.9, 1.2, 1.0 Hz, 1H), 7.91-7.82 (m, 4H), 7.47 (ddd, J=7.6, 4.6, 1.2 Hz, 1H), 7.35-7.30 (m, 3H), 5.59 (t, J=6.5 Hz, 1H), 4.32 (d, J=6.5 Hz, 2H).

### [Reference example 3]

### (6-{(Pyridin-2-ylsulfonyl)[4-(thiazol-2-yl)benzyl]aminomethyl}pyridin-2-ylamino)-acetic acid

### 3-(a) tert-Butyl [tert-butoxycarbonyl(6-{(pyridin-2-ylsulfonyl)[4-(thiazol-2-yl)benzyl]-aminomethyl}pyridin-2-yl)amino]acetate

To 6.8 ml of a tetrahydrofuran solution containing 275 mg (0.830 mmol) ofN-[4-(thiazol-2-yl)benzyl]pyridin-2-ylsulfonamide obtained in Reference example 2-(e) were added 279 mg (0.824 mmol) oftert-butyl [tert-butoxycarbonyl(6-hydroxymethylpyridin-2-yl)amino]acetate obtained in Reference example 1-(b), 308 µl (1.25 mmol) of tri-n-butylphosphine and 216 mg (1.25 mmol) of N,N,N',N'-tetramethylazodicarboxamide, and the mixture was stirred at room temperature for 12 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The obtained residue was applied to silica gel column chromatography (eluent; n-hexane: ethyl acetate=1:1 (V/V)), and the fractions containing the objective material were concentrated under reduced pressure to obtain 496 mg of the title compound as white foam. (Yield: 92%)
Mass spectrum (FAB, m/z): 652 (M⁺+1).
¹H-NMR spectrum (CDCl₃, δ ppm): 8.60 (ddd, J=4.7, 1.7, 0.9 Hz, 1H), 7.85 (d, J=3.1 Hz, 1H), 7.85-7.81 (m, 3H), 7.77 (ddd, J=7.7, 7.6, 1.7 Hz, 1H), 7.65 (d, J=8.3 Hz, 1H), 7.45 (dd, J=8.3, 7.3 Hz, 1H), 7.39 (ddd, J=7.6, 4.7, 1.3 Hz, 1H), 7.34-7.30 (m, 2H), 7.32 (d, J=3.1 Hz, 1H), 6.91 (dd, J=7.3, 0.4 Hz, 1H), 4.75 (s, 2H), 4.49 (s, 2H), 4.45 (s, 2H), 1.52 (s, 9H), 1.42 (s, 9H).

### 3-(b) (6-{(Pyridin-2-ylsulfonyl)[4-(thiazol-2-yl)benzyl]aminomethyl}pyridin-2-ylamino)acetic acid

To 4.5 ml of a methylene chloride solution containing 490 mg (0.752 mmol) of tert-butyl [tert-butoxycarbonyl(6-{(pyridin-2-ylsulfonyl)[4-(thiazol-2-yl)benzyl]aminomethyl}pyridin-2-yl)amino]acetate obtained in Reference example 3-(a) was added 3.7 ml of 4N hydrogen chloride/1,4-dioxane solution, and the mixture was stirred at room temperature for 50 hours. After completion of the reaction, the reaction mixture was concentrated under reduced pressure, to the obtained residue were added 10 ml of tetrahydrofuran, 20 ml of water, and after adjusting a pH of the mixture to 12.0 with 1N aqueous sodium hydroxide solution, the insoluble material was filtered off. 1N hydrochloric acid was added to the filtrate to adjust a pH to 4.5, and the precipitated solid was collected by filtration. The obtained solid was washed with water, and dried at 50°C under reduced pressure to obtain 147 mg of the title compound as white solid. (Yield: 39%)
Mass spectrum (FAB, m/z): 496 (M⁺+1).
¹H-NMR spectrum (DMSO-d₆, δ ppm): 12.40 (brs, 0.7H), 8.65 (ddd, J=4.6, 1.7, 0.9 Hz, 1H), 7.96 (ddd, J=7.8, 7.7, 1.7 Hz, 1H), 7.92 (d, J=3.2 Hz, 1H), 7.88-7.84 (m, 2H), 7.81 (ddd, J=7.8, 1.0, 0.9 Hz, 1H), 7.78 (d, J=3.2 Hz, 1H), 7.58 (ddd, J=7.7, 4.6, 1.0 Hz, 1H), 7.39-7.36 (m, 2H), 7.19 (dd, J=8.2, 7.1 Hz, 1H), 6.75 (t, J=5.6 Hz, 0.9H), 6.34 (d, J=8.2 Hz, 1H), 6.29 (d, J=7.1 Hz, 1H), 4.75 (s, 2H), 4.25 (s, 2H), 3.82 (d, J=5.6 Hz, 2H).
Rf value: 0.53 (n-butanol:acetic acid:water=3:1:1).

### [Reference example 4]

### (6-{[4-(Pyrazol-1-yl)benzyl](pyridin-3-ylsulfonyl)aminomethyl}pyridin-2-ylamino)-acetic acid

### 4-(a) tert-Butyl (tert-butoxycarbonyl{6-[(pyridin-3-ylsulfonyl)aminomethyl]pyridin-2-yl}amino)acetate

To 14 ml of a methylene chloride solution containing 640 mg (3.60 mmol) of 3-pyridylsulfonyl chloride were added 1.20 g (3.56 mmol) oftert-butyl [(6-aminomethylpyridin-2-yl)tert-butoxycarbonylamino]acetate obtained by the same manner as in Reference example 1-(e) and 2.24 ml (16.2 mmol) of triethylamine, and the mixture was stirred at room temperature for 1 hour. After completion of the reaction, to the reaction mixture was added a 5% aqueous potassium hydrogen sulfate solution, and the mixture was extracted with chloroform. The organic layer was successively washed with a saturated aqueous sodium hydrogen carbonate solution and a saturated aqueous sodium chloride solution, dried over anhydrous magnesium sulfate, and concentrated under reduced pressure. The obtained residue was applied to silica gel column chromatography (eluent; n-hexane:ethyl acetate=1:1→1:2 (V/V)), and the fractions containing the objective material were concentrated under reduced pressure to obtain 1.45 g of the title compound as colorless oil. (Yield: 85%)
Mass spectrum (CI, m/z): 479 (M⁺+1).
¹H-NMR spectrum (CDCl₃, δ ppm): 9.06 (d, J=2.2 Hz, 1H), 8.71 (dd, J=4.6, 1.5 Hz, 1H), 8.13-8.08 (m, 1H), 7.68 (d, J=8.2 Hz, 1H), 7.52 (dd, J=8.2, 7.4 Hz, 1H), 7.38-7.32 (m, 1H), 6.77 (d, J=7.4 Hz, 1H), 5.80 (t, J=5.1 Hz, 1H), 4.40 (s, 2H), 4.24 (d, J=5.1 Hz, 2H), 1.53 (s, 9H), 1.46 (s, 9H).

### 4-(b) tert-Butyl [tert-butoxycarbonyl(6-{(pyridin-3-ylsulfonyl)[4-(pyrazol-1-yl)benzyl]-aminomethyl}pyridin-2-yl)amino]acetate

To 65 ml of a tetrahydrofuran solution containing 5.26 g (11.0 mmol) of tert-butyl (tert-butoxycarbonyl{6-[(pyridin-3-ylsulfonyl)aminomethyl]pyridin-2-yl}amino)-acetate obtained by the same manner as in Reference example 4-(a) were added 2.00 g (11.5 mmol) of 4-(pyrazol-1-yl)benzyl alcohol (see European Journal of Medicinal Chemistry, 219, 27 (1992)), 4.0 ml (16 mmol) of tri-n-butylphosphine and 2.84 g (16.5 mmol) of N,N,N',N'-tetramethylazodicarboxamide, and the mixture was stirred at room temperature for 16 hours. After completion of the reaction, water was added to the reaction mixture, and the mixture was extracted with ethyl acetate. The organic layer was washed with a saturated sodium chloride solution, dried over anhydrous magnesium sulfate and concentrated under reduced pressure. The obtained residue was applied to silica gel column chromatography (eluent; n-hexane:ethyl acetate=1:2 (V/V)), and the fractions containing the objective material were concentrated under reduced pressure to obtain 6.57 g of the title compound as white foam. (Yield: 94%) Mass spectrum (FAB, m/z): 635 (M⁺+1).
¹H-NMR spectrum (CDCl₃, δ ppm): 8.95 (dd, J=2.3, 0.7 Hz, 1H), 8.71 (dd, J=4.9, 1.6 Hz, 1H), 7.91 (dd, J=2.5, 0.6 Hz, 1H), 7.87 (ddd, J=8.0, 2.3, 1.6 Hz, 1H), 7.72 (dd, J=1.8, 0.6 Hz, 1H), 7.71 (d, J=8.4 Hz, 1H), 7.63-7.60 (m, 2H), 7.51 (dd, J=8.4, 7.3 Hz, 1H), 7.35-7.30 (m, 3H), 6.85 (d, J=7.3 Hz, 1H), 6.47 (dd, J=2.5, 1.8 Hz, 1H), 4.61 (s, 2H), 4.39 (s, 2H), 4.35 (s, 2H), 1.53 (s, 9H), 1.42 (s, 9H).

### 4-(c) (6-{[4-(Pyrazol-1-yl)benzyl](pyridin-3-ylsulfonyl)aminomethyl}pyridin-2-yl-amino)acetic acid

To 21.5 ml of a tetrahydrofuran solution containing 6.55 g (10.3 mmol) of tert-butyl [tert-butoxycarbonyl(6-{(pyridin-3-ylsulfonyl)[4-(pyrazol-1-yl)benzyl]aminomethyl}pyridin-2-yl)amino]acetate obtained in Reference example 4-(b) were added 8.6 ml of concentrated hydrochloric acid and 21.5 ml of water, and the mixture was stirred at 65°C for 2.5 hours. After completion of the reaction, water was added to the reaction mixture, a pH of the mixture was adjusted to 4.5 with 2N aqueous sodium hydroxide solution, and the mixture was extracted with ethyl acetate. The organic layer was washed with a saturated sodium chloride solution, dried over anhydrous magnesium sulfate and concentrated under reduced pressure. A mixed solvent (ethyl acetate:diisopropyl ether=1/3 (V/V)) was added to the obtained residue, and the mixture was stirred at 50°C for 1 hour. The precipitated solid was collected by filtration, and dried under reduced pressure to obtain 4.61 g of the title compound as white solid.
(Yield: 94%)
Mass spectrum (FAB, m/z): 479 (M⁺+1).
¹H-NMR spectrum (DMSO-d₆, δ ppm): 12.42 (brs, 0.9H), 8.84 (dd, J=2.4, 0.8 Hz, 1H), 8.72 (dd, J=4.8, 1.6 Hz, 1H), 8.48 (dd, J=2.5, 0.6 Hz, 1H), 8.04 (ddd, J=8.1, 2.4, 1.6 Hz, 1H), 7.81-7.77 (m, 2H), 7.74 (dd, J=1.7, 0.6 Hz, 1H), 7.48 (ddd, J=8.1, 4.8, 0.8 Hz, 1H), 7.41-7.37 (m, 2H), 7.24 (dd, J=8.4, 7.1 Hz, 1H), 6.79 (t, J=5.9 Hz, 1H), 6.55 (dd, J=2.5, 1.7 Hz, 1H), 6.37 (dd, J=8.4, 0.6 Hz, 1H), 6.33 (dd, J=7.1, 0.6 Hz, 1H), 4.69 (s, 2H), 4.20 (s, 2H), 3.71 (d, J=5.9 Hz, 2H).
Rf value: 0.51 (n-butanol: acetic acid:water=3:1:1).

### [Test example 1]

### Measurement of EP2 receptor binding action

Measurement of EP2 receptor binding action was carried out in compliance with the method of Abramovitz et al. (Biochimica et Biophysica Acta, 1483, 285 (2000)). A test compound (a compound wherein R¹ in the compound represented by the formula (I) of the present invention is a hydroxy group) dissolved in dimethylsulfoxide and [³H]prostaglandin E₂ (NET-428, available from PerkinElmer) (final concentration: 10 nM) were added to a buffer solution (10 mM MES-KOH (pH 6.0), 10 mM MgCl₂, 1 mM EDTA) in which was suspended 10 µg of a membrane fraction of HEK293 cells expressing human EP2 receptor (ES-562-M, available from Euroscreen) followed by incubating for 60 minutes at 30°C. The membrane fraction was recovered on glass fiber filter paper (GF/B; available from Whatmann) using a cell harvester (M30R, Brandel), and after washing with a buffer solution (10 mM MES-KOH (pH 6.0), 10 mM MgCl₂), radioactivity was measured with a liquid scintillation analyzer (2000CA, Packard). The concentration of test compound required to replace 50% of the [³H]prostaglandin E₂ bound to the receptor (IC₅₀ value) was calculated using EXSAS (Ver. 7.1.6, available from Arm Systex), and the inhibition constant (Ki value) was determined using the formula indicated below.
Ki=IC₅₀/(1+([³H]prostaglandin E₂ concentration/Kd))
The dissociation constant (Kd value) was calculated by Scatchard analysis.
The test results are shown in Table 1.

**[Table 1]**

| Test compound Example No. | Ki value (nM) of EP2 Receptor Binding Action |
|---|---|
| Example 1 | 1.9 |
| Example 11 | 13 |

In this test, active forms of the compounds of the present invention demonstrated superior EP2 receptor binding action.

### [Test example 2]

### Measurement of EP2 Agonist Activity

Measurement of EP2 agonist activity was carried out in compliance with the method of Wilson et al. (European Journal of Pharmacology, 501, 49 (2004)). HEK293 cells expressing human EP2 receptor (ES-562-C, available from Euroscreen) were cultured in MEM medium containing 10% FBS and seeded at 2 x 10⁴ cells per well of a 96-well plate. On the following day, the medium was replaced with serum-free MEM medium containing 3-isobutyl-1-methylxanthine (final concentration: 500 µM) and after culturing for 30 minutes, a test compound (a compound wherein R¹ in the compound represented by the formula (I) of the present invention is a hydroxy group) dissolved in dimethylsulfoxide was added followed by allowing to stand undisturbed in a carbon dioxide incubator. After 30 minutes, the amount of cAMP in the cells was measured with a cAMP Biotrak EIA System kit (available from GE Healthcare Biosciences). The concentration of test compound required to increase the amount of cAMP to 50% of the maximum increase (EC₅₀ value) was calculated by non-linear regression of the test compound concentration and amount of cAMP using EXSAS.
The test results are shown in Table 2.

**[Table 2]**

| Test compound Example No. | Ki value (nM) of EP2 Receptor Binding Action |
|---|---|
| Example 1 | 0.45 |
| Example 11 | 2.8 |

In this test, active forms of the compounds of the present invention demonstrated superior EP2 agonist activity.

### INDUSTRIAL APPLICABILITY

Since the substituted carbonyl compound represented by the formula (1) of the present invention, or a pharmaceutically acceptable salt thereof, demonstrates superior bronchodilatory action based on potent EP2 agonistic action of its active form, while also having superior properties as a pharmaceutical composition in terms of tissue distribution, bioavailability (BA), fast-acting pharmaceutical effect, sustained pharmaceutical effect, solubility, physical stability, drug interaction, toxicity and the like, it is preferably useful as a pharmaceutical for treatment or prevention of respiratory diseases (such as asthma, COPD, bronchitis, emphysema, pulmonary fibrosis, acute respiratory distress syndrome (ARDS), cystic fibrosis and pulmonary hypertension), and moreover, is also useful as a pharmaceutical for treatment and/or prevention of diseases for which EP2 agonistic action is thought to be useful (such as bone diseases, gastric ulcer, hypertension and glaucoma).

## Claims

1. A substituted carbonyl compound represented by the formula (I): 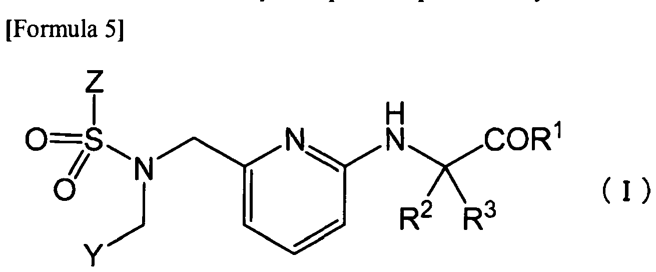 [wherein
R¹ represents either one of the following (a) to (c);
(a) a group -OR⁵ (wherein R⁵ represents a C₇-C₂₂ alkyl group; a C₇-C₁₈ aralkyl group; a halogeno-C₁-C₆ alkyl group; a C₃-C₈ cycloalkyl group which may be substituted by a group(s) selected from the group consisting of a halogeno group, an oxo group, a C₁-C₆ alkyl group, a halogeno-C₁-C₆ alkyl group and a C₁-C₅ alkylene group; or a C₁-C₆ alkyl group which is substituted by a group(s) selected from the group consisting of a C₃-C₈ cycloalkyl group, a C₂-C₆ alkanoyloxy group, an N,N-di(C₁-C₆ alkyl)aminocarbonyl group, a (C₁-C₆ alkoxy)carbonyloxy group and a 5- to 6-membered heterocyclic group.),
(b) a group -O(CH₂CH₂O)ₘR⁶ (wherein R⁶ represents a hydrogen atom or a benzyl group, and m is an integer of 1 to 4.), or,
(c) a group -NR⁷R⁸ (wherein R⁷ and R⁸ may be the same or different from each other, and each represents a hydrogen atom, a C₁-C₁₂ alkyl group, a halogeno-C₁-C₆ alkyl group or a C₃-C₈ cycloalkyl group, or, R⁷ and R⁸ may be combined to form a 4- to 8-membered ring which may be substituted by a group(s) selected from the group consisting of a halogeno group, an oxo group, a C₁-C₆ alkyl group, a halogeno-C₁-C₆ alkyl group and a C₁-C₅ alkylene group.),
R² and R³ each independently represent a hydrogen atom or a C₁-C₆ alkyl group,
Y represents either one of the following (d) to (g);
(d) a bicyclic heteroaromatic ring group which may be substituted by the same or different 1 to 5 groups selected from the group consisting of a halogeno group, a C₁-C₆ alkyl group, a halogeno-C₁-C₆ alkyl group, a C₁-C₆ alkoxy group, a halogeno-C₁-C₆ alkoxy group and a C₁-C₆ alkylthio group,
(e) a group -Q¹-Q² (wherein Q¹ represents an arylene group or a 5- to 6-membered heteroarylene group, and Q² represents an aryl group or a 5- to 6-membered heterocyclic group, each of which may be substituted by the same or different 1 to 5 groups selected from the group consisting of a halogeno group, a hydroxy group, a C₁-C₆ alkyl group, a halogeno-C₁-C₆ alkyl group, a C₁-C₆ alkoxy group and a halogeno-C₁-C₆ alkoxy group.),
(f) an aryl group or a 5- to 6-membered heterocyclic group which is substituted by a group represented by the formula (II): 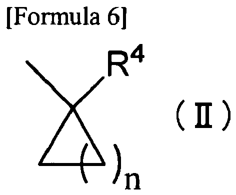 [wherein R⁴ represents a C₁-C₁₂ alkyl group or a C₁-C₆ alkoxy group, and n is an integer of 1 to 4.], or
(g) an aryl group or a 5- to 6-membered heterocyclic group, each of which may be substituted by the same or different 1 to 5 groups selected from the group consisting of a C₁-C₈ alkyl group, a halogeno-C₁-C₆ alkyl group, a C₁-C₆ alkoxy group, a halogeno-C₁-C₆ alkoxy group and a C₂-C₆ alkenyl group,
Z represents an aryl group or a 5- to 6-membered heteroaromatic group, each of which may be substituted by a group(s) selected from the group consisting of a halogeno group, a C₁-C₆alkyl group, a halogeno-C₁-C₆ alkyl group, a C₁-C₆ alkoxy group and a halogeno-C₁-C₆ alkoxy group.]
or a pharmaceutically acceptable salt thereof

2. The substituted carbonyl compound or a pharmaceutically acceptable salt thereof according to Claim 1, wherein R² and R³ each independently represent a hydrogen atom or a C₁-C₄ alkyl group.

3. The substituted carbonyl compound or a pharmaceutically acceptable salt thereof according to Claim 1 or 2, wherein R¹ represents a group -OR⁵ (wherein R⁵ represents a C₇-C₂₂ alkyl group; a C₇-C₁₈ aralkyl group; a halogeno-C₁-C₄ alkyl group; a C₃-C₆ cycloalkyl group which may be substituted by a group(s) selected from the group consisting of a halogeno group, a C₁-C₄ alkyl group, a halogeno-C₁-C₄ alkyl group and a C₁-C₃ alkylene group; or a C₁-C₆ alkyl group which is substituted by a group(s) selected from the group consisting of a C₃-C₆ cycloalkyl group, a C₂-C₄ alkanoyloxy group, an N,N-di(C₁-C₄ alkyl)aminocarbonyl group, a (C₁-C₄ alkoxy)carbonyloxy group and a 5- to 6-membered heterocyclic group.), a group -O(CH₂CH₂O)ₘR⁶ (wherein R⁶ represents a hydrogen atom or a benzyl group, and m is an integer of 3 to 4.), or, a group -NR⁷R⁸ (wherein R⁷ and R⁸ may be the same or different from each other, and each represents a hydrogen atom, a C₁-C₁₂ alkyl group, a halogeno-C₁-C₄ alkyl group or a C₃-C₆ cycloalkyl group, or, R⁷ and R⁸ may form a 4- to 6-membered ring in combination which may be substituted by a group(s) selected from the group consisting of a halogeno group, a C₁-C₄ alkyl group, a halogeno-C₁-C₄ alkyl group and a C₁-C₃ alkylene group.).

4. The substituted carbonyl compound or a pharmaceutically acceptable salt thereof according to any one of Claims 1 to 3, wherein Y represents a benzofuryl group, a benzothienyl group, a benzoxazolyl group or a benzothiazolyl group, each of which may be substituted by a group(s) selected from the group consisting of a halogeno group, a C₁-C₄ alkyl group, a halogeno-C₁-C₄ alkyl group, a C₁-C₄ alkoxy group, a halogeno-C₁-C₄ alkoxy group and a C₁-C₄ alkylthio group, or, a group -Q¹-Q²(Q¹ represents a phenylene group, a thienylene group, a pyridazinylene group or a pyrimidinylene group, Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group, each of which may be substituted by a group(s) selected from the group consisting of a halogeno group, a hydroxy group, a C₁-C₄ alkyl group, a halogeno-C₁-C₄ alkyl group, a C₁-C₄ alkoxy group and a halogeno-C₁-C₄ alkoxy group), or, a phenyl group which is substituted by the following formula (II): 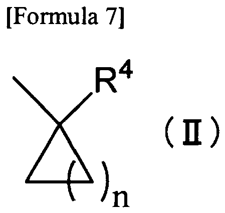 (R⁴ is a C₁-C₁₀ alkyl group or a C₁-C₄ alkoxy group, and n is an integer of 1 to 2), or, a phenyl group or a thienyl group, each of which may be substituted by a group(s) selected from the group consisting of a C₃-C₆ alkyl group, a halogeno-C₁-C₄ alkyl group, a C₁-C₄ alkoxy group, a halogeno-C₁-C₄ alkoxy group and a C₄-C₆ alkenyl group.

5. The substituted carbonyl compound or a pharmaceutically acceptable salt thereof according to any one of Claims 1 to 4, wherein Z represents a phenyl group, a thienyl group, an imidazolyl group, a thiazolyl group, a pyridyl group or a pyrimidinyl group, each of which may be substituted by a group(s) selected from the group consisting of a halogeno group, a C₁-C₄ alkyl group, a halogeno-C₁-C₄ alkyl group, a C₁-C₄ alkoxy group and a halogeno-C₁-C₄ alkoxy group.

6. The substituted carbonyl compound or a pharmaceutically acceptable salt thereof according to any one of Claims 1 to 5, wherein R¹ represents a group -OR⁵ (wherein R⁵ represents a C₇-C₂₂ alkyl group; a C₈-C₁₈ aralkyl group; a fluoro-C₁-C₄ alkyl group; a C₃-C₆ cycloalkyl group which may be substituted by a group(s) selected from the group consisting of a fluoro group, a methyl group and a trifluoromethyl group; or a C₁-C₄ alkyl group which is substituted by a group(s) selected from the group consisting of a cyclopropyl group, an acetoxy group, a pivaloyloxy group, an N,N-di(C₁-C₄ alkyl)-aminocarbonyl group, a methoxycarbonyloxy group, a morpholinyl group, a 5-methyl-2-oxo-1,3-dioxolen-4-yl group and a 5-phenyl-2-oxo-1,3-dioxolen-4-yl group.), a group -O(CH₂CH₂O)ₘR⁶ (wherein R⁶ represents a hydrogen atom or a benzyl group, and m is an integer of 3 to 4.), or, a group -NR⁷R⁸ (wherein R⁷ and R⁸ may be the same or different from each other, and each represents a hydrogen atom, a C₁-C₁₁ alkyl group, a fluoro-C₁-C₄ alkyl group or a C₃-C₆ cycloalkyl group.).

7. The substituted carbonyl compound or a pharmaceutically acceptable salt thereof according to Claim 6, wherein R¹ represents a heptyloxy group, an octyloxy group, a nonyloxy group, a decyloxy group, an undecyloxy group, a dodecyloxy group, a tridecyloxy group, a tetradecyloxy group, a pentadecyloxy group, a cetyloxy group, a heptadecyloxy group, an octadecyloxy group, a nonadecyloxy group, an eicosyloxy group, a heneicosyloxy group, a docosyloxy group, a 4-phenylbutyloxy group, a 5-phenylpentyloxy group, a 6-phenylhexyloxy group, a 7-phenylheptyloxy group, a 8-phenyloctyloxy group, a 9-phenylnonyloxy group, a 10-phenyldecyloxy group, a 11-phenylundecyloxy group, a 12-phenyldodecyloxy group, a 2,2,2-trifluoroethoxy group, a cyclohexyloxy group, an acetoxymethoxy group, a pivaloyloxymethoxy group, a 2-(dimethylamino)-2-oxoethoxy group, a 2-(diethylamino)-2-oxoethoxy group, a (5-methyl-2-oxo-1,3-dioxolen-4-yl)methoxy group, a (5-phenyl-2-oxo-1,3-dioxolen-4-yl)methoxy group, a (morpholin-4-yl)methoxy group, a 2-(morpholin-4-yl)ethoxy group, a 2-[2-(2-hydroxyethoxy)ethoxy]ethoxy group, a 2-{2-[2-(benzyloxy)ethoxy]-ethoxy}ethoxy group, a methylamino group, an ethylamino group, a propylamino group, an isopropylamino group, a butylamino group, a hexylamino group, an undecylamino group, a dimethylamino group, a diethylamino group or a (2,2,2-trifluoroethyl)amino group.

8. The substituted carbonyl compound or a pharmaceutically acceptable salt thereof according to Claim 4, wherein Y represents a benzofuryl group, a benzothienyl group, a benzoxazolyl group or a benzothiazolyl group, each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group, a dichloromethoxy group, a methylthio group, an ethylthio group, a propylthio group, an isopropylthio group and a tert-butylthio group, or, a group -Q¹-Q² (wherein Q¹ represents a phenylene group, a thienylene group, a pyridazinylene group or a pyrimidinylene group, Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group, each of which may be substituted by a group(s) selected from the group consisting of a fluoro group, a chloro group, a bromo group, a hydroxy group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group and a dichloromethoxy group), or, a phenyl group which is substituted by a group(s) selected from the group consisting of a 1-methylcyclopropyl group, a 1-ethylcyclopropyl group, a 1-isopropylcyclopropyl group, a 1-butylcyclopropyl group, a 1-hexylcyclopropyl group, a 1-methoxycyclopropyl group and a 1-ethylcyclobutyl group, or, a phenyl group which may be substituted by a group(s) selected from the group consisting of a tert-butyl group, a neopentyl group, a tert-pentyl group, a 1-ethylpropyl group, a 1-ethyl-1-methylpropyl group, a trifluoromethyl group, a difluoromethoxy group and a 1-methyl-1-pentenyl group.

9. The substituted carbonyl compound or a pharmaceutically acceptable salt thereof according to Claim 5, wherein Z represents a phenyl group, a thienyl group or a pyridyl group, each of which may be substituted by a group(s) selected from the group consisting of a fluoro group, a chloro group, a methyl group, an ethyl group, a trifluoromethyl group, a methoxy group and a difluoromethoxy group.

10. The substituted carbonyl compound or a pharmaceutically acceptable salt thereof according to Claim 1, wherein
R¹ is a group -OR⁵ (wherein R⁵ represents a C₇-C₂₂ alkyl group; a C₈-C₁₈ aralkyl group; a fluoro-C₁-C₄ alkyl group; a C₃-C₆ cycloalkyl group which may be substituted by a group(s) selected from the group consisting of a fluoro group, a methyl group and a trifluoromethyl group; or a C₁-C₄ alkyl group which is substituted by a group(s) selected from the group consisting of a cyclopropyl group, an acetoxy group, a pivaloyloxy group, an N,N-di(C₁-C₄ alkyl)aminocarbonyl group, a methoxycarbonyloxy group, a morpholinyl group, a 5-methyl-2-oxo-1,3-dioxolen-4-yl group and a 5-phenyl-2-oxo-1,3-dioxolen-4-yl group.), a group -O(CH₂CH₂O)ₘR⁶ (wherein R⁶ represents a hydrogen atom or a benzyl group, and m is an integer of 3 to 4.), or, a group -NR⁷R⁸ (wherein R⁷ and R⁸ may be the same or different from each other, and each represents a hydrogen atom, a C₁-C₁₁ alkyl group, a fluoro-C₁-C₄ alkyl group or a C₃-C₆ cycloalkyl group.),
R² and R³ each independently represent a hydrogen atom or methyl group,
Y is a benzofuryl group, a benzothienyl group, a benzoxazolyl group or a benzothiazolyl group, each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group, a dichloromethoxy group, a methylthio group, an ethylthio group, a propylthio group, an isopropylthio group and a tert-butylthio group, or, a group -Q¹-Q² (wherein Q¹ represents a phenylene group, a thienylene group, a pyridazinylene group or a pyrimidinylene group, and Q² represents a phenyl group, a thienyl group, a pyrazolyl group, an oxazolyl group, a thiazolyl group, a 1,2,4-triazolyl group, a pyridyl group, a pyridazinyl group, a pyrimidinyl group, a 4,5-dihydrothiazolyl group, a pyrrolidinyl group or a piperidinyl group, each of which may be substituted by a group(s) selected from the group consisting of a fluoro group, a chloro group, a bromo group, a hydroxy group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a tert-butoxy group, a trifluoromethoxy group, a difluoromethoxy group, a trichloromethoxy group and a dichloromethoxy group), or, a phenyl group which is substituted by a group(s) selected from the group consisting of a 1-methylcyclopropyl group, a 1-ethylcyclopropyl group, a 1-isopropylcyclopropyl group, a 1-butylcyclopropyl group, a 1-hexylcyclopropyl group, a 1-methoxycyclopropyl group and a 1-ethylcyclobutyl group, or, a phenyl group which may be substituted by a group(s) selected from the group consisting of a tert-butyl group, a neopentyl group, a tert-pentyl group, a 1-ethylpropyl group, a 1-ethyl-1-methylpropyl group, a trifluoromethyl group, a difluoromethoxy group and a 1-methyl-1-pentenyl group,
Z represents a phenyl group, a thienyl group or a pyridyl group, each of which may be substituted by a group(s) selected from the group consisting of a fluoro group, a chloro group, a methyl group, an ethyl group, a trifluoromethyl group, a methoxy group and a difluoromethoxy group.

11. The substituted carbonyl compound or a pharmaceutically acceptable salt thereof according to Claim 1, wherein
R¹ is a heptyloxy group, an octyloxy group, a nonyloxy group, a decyloxy group, an undecyloxy group, a dodecyloxy group, a tridecyloxy group, a tetradecyloxy group, a pentadecyloxy group, a cetyloxy group, a heptadecyloxy group, an octadecyloxy group, a nonadecyloxy group, an eicosyloxy group, a heneicosyloxy group, a docosyloxy group, a 4-phenylbutyloxy group, a 5-phenylpentyloxy group, a 6-phenylhexyloxy group, a 7-phenylheptyloxy group, a 8-phenyloctyloxy group, a 9-phenylnonyloxy group, a 10-phenyldecyloxy group, a 11-phenylundecyloxy group, a 12-phenyldodecyloxy group, a 2,2,2-trifluoroethoxy group, a cyclohexyloxy group, an acetoxymethoxy group, a pivaloyloxymethoxy group, a 2-(dimethylamino)-2-oxoethoxy group, a 2-(diethylamino)-2-oxoethoxy group, a (5-methyl-2-oxo-1,3-dioxolen-4-yl)-methoxy group, a (5-phenyl-2-oxo-1,3-dioxolen-4-yl)methoxy group, a (morpholin-4-yl)methoxy group, a 2-(morpholin-4-yl)ethoxy group, a 2-[2-(2-hydroxyethoxy)-ethoxy]ethoxy group, a 2-{2-[2-(benzyloxy)ethoxy]ethoxy}ethoxy group, a methylamino group, an ethylamino group, a propylamino group, an isopropylamino group, a butylamino group, a hexylamino group, an undecylamino group, a dimethylamino group, a diethylamino group or a (2,2,2-trifluoroethyl)amino group,
R² and R³ are both hydrogen atoms,
Y is a benzofuran-2-yl group, a benzo[b]thiophen-2-yl group, a 6-chlorobenzo-[b]thiophen-2-yl group, a 6-methoxybenzo[b]thiophen-2-yl group, a biphenyl-4-yl group, a 4'-fluorobiphenyl-4-yl group, a 4'-chlorobiphenyl-4-yl group, a 4-(pyrazol-1-yl)phenyl group, a 4-(thiazol-2-yl)phenyl group, a 4-(5-chlorothiazol-2-yl)phenyl group, a 4-(5-methylthiazol-2-yl)phenyl group, a 4-(4,5-dimethylthiazol-2-yl)phenyl group, a 4-(4-trifluoromethylthiazol-2-yl)phenyl group, a 4-(thiazol-4-yl)phenyl group, a 4-(1,2,4-triazol-1-yl)phenyl group, a 4-(pyridin-2-yl)phenyl group, a 4-(pyridazin-4-yl)-phenyl group, a 4-(pyrimidin-2-yl)phenyl group, a 4-(4,5-dihydrothiazol-2-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-hexylcyclopropyl)phenyl group, a 4-(1-methoxycyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)-phenyl group, a 4-(1-ethyl-1-methylpropyl)phenyl group, a 4-trifluoromethylphenyl group, a 4-difluoromethoxyphenyl group or a 4-(1-methyl-1-pentenyl)phenyl group, and
Z represents a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-ethylphenyl group, a 4-ethyl-3-fluorophenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a thiophen-2-yl group, a thiophen-3-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group.

12. The substituted carbonyl compound or a pharmaceutically acceptable salt thereof according to Claim 1, wherein
R¹ is an undecyloxy group, a docosyloxy group, a 5-phenylpentyloxy group, a 10-phenyldecyloxy group, a 2,2,2-trifluoroethoxy group, a 2-(dimethylamino)-2-oxo-ethoxy group, a pivaloyloxymethoxy group, a (5-methyl-2-oxo-1,3-dioxolen-4-yl)-methoxy group, a 2-(morpholin-4-yl)ethoxy group, a 2-[2-(2-hydroxyethoxy)ethoxy]-ethoxy group, a 2-{2-[2-(benzyloxy)ethoxy]ethoxy}ethoxy group, an undecylamino group or a dimethylamino group,
R² and R³ are both hydrogen atoms,
Y is a benzofuran-2-yl group, a benzo[b]thiophen-2-yl group, a 6-chlorobenzo-[b]thiophen-2-yl group, a 6-methoxybenzo[b]thiophen-2-yl group, a biphenyl-4-yl group, a 4'-fluorobiphenyl-4-yl group, a 4-(pyrazol-1-yl)phenyl group, a 4-(thiazol-2-yl)phenyl group, a 4-(thiazol-4-yl)phenyl group, a 6-phenylpyridazin-3-yl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-ethylcyclobutyl)phenyl group, a 4-(tert-butyl)phenyl group, a 4-neopentylphenyl group, a 4-(tert-pentyl)phenyl group, a 4-(1-ethylpropyl)phenyl group or a 4-(1-ethyl-1-methylpropyl)phenyl group, and
Z represents a phenyl group, a 3-fluorophenyl group, a 4-fluorophenyl group, a pyridin-2-yl group, a pyridin-3-yl group or a pyridin-4-yl group.

13. The substituted carbonyl compound or a pharmaceutically acceptable salt thereof according to Claim 1, wherein the substituted carbonyl compound is
(5-phenylpentyl) (6-{(pyridin-2-ylsulfonyl)[4-(thiazol-2-yl)benzyl]aminomethyl}-pyridin-2-ylamino)acetate,
(10-phenyldecyl) (6-{(pyridin-2-ylsulfonyl)[4-(thiazol-2-yl)benzyl]aminomethyl}-pyridin-2-ylamino)acetate,
2-(6-{(pyridin-2-ylsulfonyl)[4-(thiazol-2-yl)benzyl]aminomethyl}pyridin-2-ylamino)-N-undecylacetamide,
N,N-dimethyl-2-(6-{(pyridin-2-ylsulfonyl)[4-(thiazol-2-yl)benzyl]aminomethyl}-pyridin-2-ylamino)acetamide,
(2-{2-[2-(benzyloxy)ethoxy]ethoxy}ethyl)(6-{(pyridin-2-ylsulfonyl)[4-(thiazol-2-yl)-benzyl]aminomethyl}pyridin-2-ylamino)acetate,
{2-[2-(2-hydroxyethoxy)ethoxy]ethyl}(6-{(pyridin-2-ylsulfonyl)[4-(thiazol-2-yl)-benzyl]aminomethyl}pyridin-2-ylamino)acetate,
[(5-methyl-2-oxo-1,3-dioxolen-4-yl)methyl](6-{(pyridin-2-ylsulfonyl)[4-(thiazol-2-yl)-benzyl]aminomethyl}pyridin-2-ylamino)acetate,
pivaloyloxymethyl (6-{(pyridin-2-ylsulfonyl)[4-(thiazol-2-yl)benzyl]aminomethyl}-pyridin-2-ylamino)acetate,
[2-(dimethylamino)-2-oxoethyl] (6-{(pyridin-2-ylsulfonyl)[4-(thiazol-2-yl)benzyl]-aminomethyl} pyridin-2-ylamino)acetate,
[2-(morpholin-4-yl)ethyl] (6-{(pyridin-2-ylsulfonyl)[4-(thiazol-2-yl)benzyl]aminomethyl}pyridin-2-ylamino)acetate,
(2,2,2-trifluoroethyl) (6-{[4-(pyrazol-1-yl)benzyl](pyridin-3-ylsulfonyl)aminomethyl}-pyridin-2-ylamino)acetate,
docosyl (6-{(pyridin-2-ylsulfonyl)[4-(thiazol-2-yl)benzyl]aminomethyl}pyridin-2-yl-amino)acetate, or
undecyl (6-{(pyridin-2-ylsulfonyl)[4-(thiazol-2-yl)benzyl]aminomethyl}pyridin-2-yl-amino)acetate.

14. A pharmaceutical composition comprising the substituted carbonyl compound according to any one of claims 1 to 13 or a pharmaceutically acceptable salt thereof as an active ingredient.

15. The pharmaceutical composition according to claim 14 for preventing or treating a respiratory disease.
